# EUROPEAN PATENT APPLICATION

(11) **EP 2 455 052 A1**
(43) Date of publication of application: **23.05.2012**
(21) Application number: 09847323.4
(22) Date of filing: 14.07.2009
(51) Int. Cl.: A61G 10/00, A61H 33/02, A61H 33/12

(54) **DEVICE FOR PERCUTANEOUS ABSORPTION OF CARBON DIOXIDE GAS, METHOD FOR PERCUTANEOUS ABSORPTION OF CARBON DIOXIDE GAS, AND ENVELOPE**

(71) Applicant: amuse company LTD., Tokyo 173-0027 (JP)
(72) Inventor: MOGI, Kuninobu, Tokyo 179-0072 (JP)
(74) Representative: Vossius & Partner
(86) International application number: PCT/JP2009/062759
(87) International publication number: WO 2011/007425

(57) **Abstract**

By providing an envelope body (101) enveloping at least a part of body surface, a supply unit (61) supplying carbon dioxide gas into the envelope body (101), and a pressurizing unit (102) pressurizing the carbon dioxide gas supplied into the envelope body (101) to make the gas to be absorbed into an absorbent material, it is possible to further improve an efficiency for making the carbon dioxide gas to be absorbed from the body surface.

## Description

### TECHNICAL FIELD

The present invention relates to a device for percutaneous absorption of carbon dioxide gas, a method for percutaneous absorption of carbon dioxide gas, and an envelope body with which percutaneous absorption (absorption from body surface) of carbon dioxide gas can be realized.

### BACKGROUND ART

When carbon dioxide gas (hereinafter, explanation will be made by citing carbon dioxide as carbon dioxide gas as an example) is absorbed into a body, a partial pressure of carbon dioxide in a cell in the body is increased. When the partial pressure of carbon dioxide is increased, oxygen is discharged into a cell from blood, resulting in that a partial pressure of oxygen in the cell is increased. Further, when the partial pressure of oxygen in cells in the body is increased, it is possible to provide effects such that respective cells are activated, blood vessels are dilated, a blood circulation is increased, and a blood pressure is reduced. Such effects are called as Bohr effects, which have been conventionally utilized in cosmetic and medical fields.

Here, as a method for percutaneous absorption of carbon dioxide, a technique disclosed in Patent Literature 1 has been known. The technique disclosed in Patent Literature 1 is structured by a sealing envelope member capable of enveloping a part of body, a supply unit supplying carbon dioxide into the sealing envelope member, and an absorption aid assisting percutaneous and transmucosal absorption of carbon dioxide inside the sealing envelope member. When the percutaneous absorption of carbon dioxide is performed, at first, the absorption aid being a nonwoven fabric made of polypropylene soaked in a citric acid aqueous solution, for example, is put on a part of body. Next, the part of the body on which the absorption aid is put is covered by the sealing envelope member. Further, a vinyl tube connected from a carbon dioxide blow-out port of the supply unit of carbon dioxide is inserted into the sealing envelope member, and then an opening of the sealing envelope member is tied to be sealed. The supply unit of carbon dioxide fills the inside of the sealing envelope member with carbon dioxide. The filled carbon dioxide is absorbed from a body surface through the absorption aid. As above, in the technique disclosed in Patent Literature 1, by making the carbon dioxide sealed inside of the sealing envelope member to be absorbed from the body surface through the absorption aid, it is possible to efficiently supply the carbon dioxide into the body.

### CITATION LIST

### PATENT LITERATURE

Patent Literature 1: International Publication Pamphlet No. WO 2004/002393

### SUMMARY OF INVENTION

### TECHNICAL PROBLEM

However, in the technique disclosed in Patent Literature 1 described above, the absorption aid plays a role of realizing efficient absorption of carbon dioxide, so that it is not possible to deal with a case where the efficiency of the absorption of carbon dioxide is tried to be further improved.
The present invention has been made in view of the problems as described above, and an object thereof is to enable a further improvement in efficiency for making carbon dioxide to be absorbed from a body surface.

### SOLUTION TO PROBLEM

A device for percutaneous absorption of carbon dioxide gas of the present invention is **characterized in that** it includes: an envelope body enveloping at least a part of body surface; a supply unit supplying carbon dioxide gas into the envelope body; and a pressurizing unit pressurizing the carbon dioxide gas supplied into the envelope body to make the gas to be absorbed into an absorbent material.
Further, a method for percutaneous absorption of carbon dioxide gas of the present invention is **characterized in that** it includes: enveloping at least a part of body surface using an envelope body; supplying carbon dioxide gas into the envelope body; and pressurizing the carbon dioxide gas supplied into the envelope body to make the gas to be absorbed into an absorbent material.
Further, an envelope body of the present invention being an envelope body enveloping at least a part of body surface, **characterized in that** it includes a supply space in which carbon dioxide gas is supplied to an inside thereof, in which the envelope body has an adhesiveness at least at a contact portion which is brought into contact with the body surface, and is formed of a material which is resistant to a pressure applied to the carbon dioxide gas supplied to the inside, from the inside or an outside.

### ADVANTAGEOUS EFFECTS OF INVENTION

According to the present invention, it is possible to further improve efficiency for making carbon dioxide to be absorbed from a body surface.

### BRIEF DESCRIPTION OF DRAWINGS

[Fig. 1] Fig. 1 is a diagram illustrating a structure of a carbon dioxide supply device according to a first embodiment;
[Fig. 2A] Fig. 2A is a diagram for explaining an attachment method of an envelope body;
[Fig. 2B] Fig. 2B is a diagram for explaining the attachment method of the envelope body;
[Fig. 3A] Fig. 3A is a diagram for explaining a method of supplying carbon dioxide into the envelope body attached to an arm;
[Fig. 3B] Fig. 3B is a diagram for explaining the method of supplying carbon dioxide into the envelope body attached to the arm;
[Fig. 3C] Fig. 3C is a diagram for explaining the method of supplying carbon dioxide into the envelope body attached to the arm;
[Fig. 3D] Fig. 3D is a diagram for explaining the method of supplying carbon dioxide into the envelope body attached to the arm;
[Fig. 4] Fig. 4 is a diagram illustrating a cross section of the envelope body attached to the arm;
[Fig. 5A] Fig. 5A is a diagram illustrating an envelope body of another form used in the first embodiment;
[Fig. 5B] Fig. 5B is a diagram illustrating the envelope body of the another form used in the first embodiment;
[Fig. 5C] Fig. 5C is a diagram illustrating the envelope body of the another form used in the first embodiment;
[Fig. 6A] Fig. 6A is a diagram illustrating an envelope body of another form used in the first embodiment;
[Fig. 6B] Fig. 6B is a diagram illustrating an envelope body of another form used in the first embodiment;
[Fig. 6C] Fig. 6C is a diagram illustrating an envelope body of another form used in the first embodiment;
[Fig. 7] Fig. 7 is a diagram illustrating a structure of a carbon dioxide supply device according to a second embodiment;
[Fig. 8A] Fig. 8A is a diagram illustrating a structure of a sheet-shaped absorbent material;
[Fig. 8B] Fig. 8B is a diagram illustrating a structure of the sheet-shaped absorbent material;
[Fig. 9A] Fig. 9A is a diagram illustrating an envelope body of another form used in the second embodiment;
[Fig. 9B] Fig. 9B is a diagram illustrating an envelope body of another form used in the second embodiment;
[Fig. 9C] Fig. 9C is a diagram illustrating an envelope body of another form used in the second embodiment;
[Fig. 9D] Fig. 9D is a diagram illustrating an envelope body of another form used in the second embodiment;
[Fig. 10A] Fig. 10A is a diagram illustrating a structure of a carbon dioxide supply device according to a third embodiment;
[Fig. 10B] Fig. 10B is a diagram illustrating a structure of a carbon dioxide supply device according to the third embodiment;
[Fig. 11] Fig. 11 is a diagram illustrating a structure of a sheet-shaped partition member;
[Fig. 12] Fig. 12 is a diagram illustrating a structure of a carbon dioxide supply device according to a fourth embodiment;
[Fig. 13A] Fig. 13A is a diagram for explaining an absorbent material suitable in a case where a body is enveloped by an envelope body for upper half of body;
[Fig. 13B] Fig. 13B is a diagram for explaining an absorbent material suitable in a case where a body is enveloped by an envelope body for upper half of body;
[Fig. 13C] Fig. 13C is a diagram for explaining the absorbent material suitable in a case where the body is enveloped by the envelope body for upper half of body;
[Fig. 14] Fig. 14 is a diagram illustrating an envelope body of another form used in the fourth embodiment;
[Fig. 15] Fig. 15 is a diagram illustrating an envelope body of another form used in the fourth embodiment;
[Fig. 16A] Fig. 16A is a diagram illustrating the envelope body of the another form used in the fourth embodiment;
[Fig. 16B] Fig. 16B is a diagram illustrating the envelope body of the another form used in the fourth embodiment;
[Fig. 17A] Fig. 17A is a diagram illustrating an envelope body of another form used in the fourth embodiment;
[Fig. 17B] Fig. 17B is a diagram illustrating the envelope body of the another form used in the fourth embodiment;
[Fig. 18A] Fig. 18A is a diagram illustrating a structure of an envelope body having an integrally attached absorbent body and used in a fifth embodiment;
[Fig. 18B] Fig. 18B is a diagram illustrating a structure of the envelope body having the integrally attached absorbent body and used in the fifth embodiment;
[Fig. 19A] Fig. 19A is a diagram illustrating a structure of the envelope body;
[Fig. 19B] Fig. 19B is a diagram illustrating a structure of the envelope body;
[Fig. 20A] Fig. 20A is a diagram illustrating a structure of the absorbent body;
[Fig. 20B] Fig. 20B is a diagram illustrating a structure of the absorbent body;
[Fig. 21] Fig. 21 is a diagram for explaining a method of supplying carbon dioxide from a body surface using the envelope body;
[Fig. 22A] Fig. 22A is a diagram illustrating an envelope body of another form used in the fifth embodiment;
[Fig. 22B] Fig. 22B is a diagram illustrating an envelope body of another form used in the fifth embodiment;
[Fig. 22C] Fig. 22C is a diagram illustrating an envelope body of another form used in the fifth embodiment;
[Fig. 23A] Fig. 23A is a diagram illustrating a structure of an envelope body according to a sixth embodiment;
[Fig. 23B] Fig. 23B is a diagram illustrating a structure of the envelope body according to the sixth embodiment;
[Fig. 24A] Fig. 24A is a diagram for explaining a method of supplying carbon dioxide into the envelope body put on an elbow;
[Fig. 24B] Fig. 24B is a diagram for explaining the method of supplying carbon dioxide into the envelope body put on the elbow;
[Fig. 24C] Fig. 24C is a diagram for explaining the method of supplying carbon dioxide into the envelope body put on the elbow;
[Fig. 24D] Fig. 24D is a diagram for explaining the method of supplying carbon dioxide into the envelope body put on the elbow;
[Fig. 24E] Fig. 24E is a diagram for explaining the method of supplying carbon dioxide into the envelope body put on the elbow;
[Fig. 25] Fig. 25 is a diagram illustrating a structure of a carbon dioxide supply device according to a seventh embodiment;
[Fig. 26A] Fig. 26A is a diagram for explaining a method of supplying carbon dioxide into an envelope body attached to an arm;
[Fig. 26B] Fig. 26B is a diagram for explaining the method of supplying carbon dioxide into the envelope body attached to the arm;
[Fig. 26C] Fig. 26C is a diagram for explaining the method of supplying carbon dioxide into the envelope body attached to the arm;
[Fig. 27] Fig. 27 is a diagram illustrating a structure of a concentration adjusting unit according to an eighth embodiment;
[Fig. 28A] Fig. 28A is an exterior view of the concentration adjusting unit according to the eighth embodiment;
[Fig. 28B] Fig. 28B is an exterior view of the concentration adjusting unit according to the eighth embodiment;
[Fig. 29] Fig. 29 is a diagram illustrating a structure of a concentration adjusting unit according to a ninth embodiment;
[Fig. 30] Fig. 30 is an exterior view of the concentration adjusting unit according to the ninth embodiment;
[Fig. 31A] Fig. 31A is a diagram illustrating a structure of a carbon dioxide supply device according to a tenth embodiment; and
[Fig. 31B] Fig. 31B is a diagram illustrating a structure of an envelope body according to the tenth embodiment.

### DESCRIPTION OF EMBODIMENTS

Hereinafter, a structure of a device for percutaneous absorption of carbon dioxide gas (referred to as carbon dioxide supply device, hereinafter) according to an embodiment of the present invention will be described with reference to the drawings. Note that explanation will be made by citing carbon dioxide as the carbon dioxide gas, as an example.

### (First Embodiment)

A first embodiment is an embodiment of a case where a supply unit supplying carbon dioxide into an envelope body enveloping at least a part of body surface and a pressuring unit pressurizing the carbon dioxide supplied into the envelope body to make the carbon dioxide to be absorbed into an absorbent material, are made common.

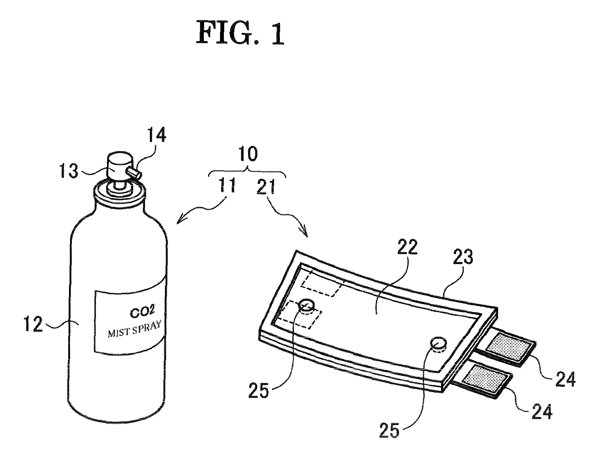

Fig. 1 illustrates a structure of a carbon dioxide supply device 10 according to the first embodiment. As illustrated in Fig. 1, the carbon dioxide supply device 10 includes a supply unit 11 and an envelope body 21.

The supply unit 11 includes a container body 12, a push-down portion 13, and a blow-out port 14. The supply unit 11 is a so-called spray can, and has a characteristic that it is portable due to its light weight. In the container body 12 of the supply unit 11 of the present embodiment, carbon dioxide to be supplied into a body and an absorbent material assisting for making the carbon dioxide to be absorbed into the body from a body surface, are accommodated in a highly compressed state. In the supply unit 11 of the present embodiment, there is accommodated carbon dioxide of about 2 to 6 liters. Here, the absorbent material is a medium in which carbon dioxide can be dissolved, and is water, alcohols, oils and fats or the like, for example. Further, the absorbent material is preferably structured such that when it is adhered to a surface of a body, the adhered absorbent material is easily remained at the adhered position. Further, the absorbent material is slightly acidic with PH of 4.0 to 6.5 for reducing an influence on the body surface, and glycerin or Vaseline may be added to the absorbent material for moisturizing and protecting the body surface. When a user pushes the push-down portion 13 of the supply unit 11, the carbon dioxide and the absorbent material mixed and accommodated in the container body 12 can be vigorously discharged from the blow-out port 14. Here, the absorbent material is preferably a nano-sized absorbent material such as nano-sized water which is easily absorbed into the body from the body surface.

The envelope body 21 includes an envelope member 22, a contact portion 23, locking portions 24, and suction ports 25. The envelope body 21 of the present embodiment is a member that envelops at least a part of body surface so as to wrap around the part.
The envelope body 21 illustrated in Fig. 1 is illustrated so that a surface with which the body surface is brought into contact can be seen.
The envelope member 22 is formed in a sheet shape. As a material of the envelope member 22 of the present embodiment, a chloroprene rubber is used, for example. The chloroprene rubber has elasticity, adhesiveness, flexibility, durability and sealing property. Further, to a back side of the chloroprene rubber used for the envelope member 22, namely, to a surface on the opposite side of the surface with which the body surface is brought into contact, a nylon jersey is bonded, thereby further enhancing the durability of the envelope member 22. Note that the material of the envelope member 22 is not limited to the chloroprene rubber, and it is also possible to use latex being a material similar to the chloroprene rubber, soft urethane, ethylene-vinyl acetate, polyvinyl chloride, a silicon rubber and the like.

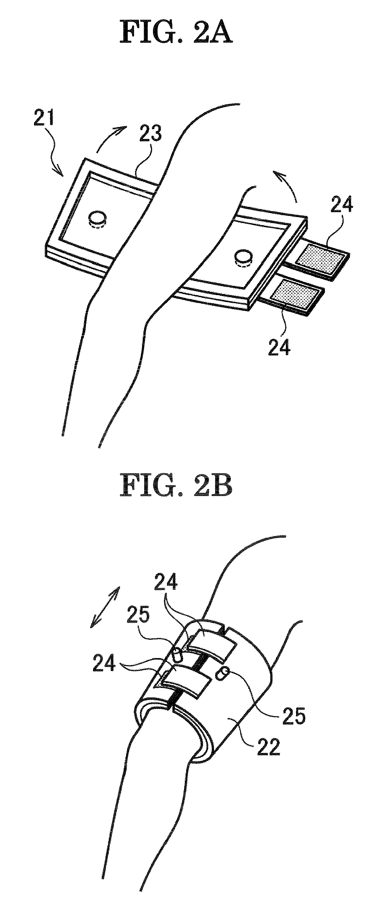

The contact portion 23 is a portion which is brought into contact with the body surface, and is standingly provided along an outer periphery of the envelope member 22. For the contact portion 23, a material having sealing property is used, and, for example, a chloroprene rubber is used. Here, by enveloping at least a part of the body surface so as to wrap around the part using the envelope body 21, there is formed, in a space surrounded by the envelope member 22, the contact portion 23 and the body surface, a supply space to which carbon dioxide is supplied.
There are provided a pair of locking portions 24 to one end portion of the envelope member 22 and to the other end portion opposite to the one end portion. In the present embodiment, two pairs of locking portions 24 are provided. Detailed explanation of the locking portion 24 will be made later in explanation of Fig. 2.
The suction port 25 has a hole communicated with the aforementioned supply space, and through the hole, carbon dioxide can be supplied to the supply space. To the envelope body 21 illustrated in Fig. 1, a plurality of (two) suction ports 25 are provided so that carbon dioxide can be uniformly supplied into the supply space. Further, in order to prevent the carbon dioxide supplied to the supply space from being discharged from the suction port 25, the suction port 25 serves as a check valve.

Next, an attachment method of the envelope body 21 will be described with reference to Fig. 2A, Fig. 2B. Here, explanation will be made by citing an arm as a region of body around which the envelope body 21 is wrapped, as an example. First, as illustrated in Fig. 2A, a user makes the surface having the contact portion 23 face the body surface side, and applies the envelope body 21 to a region of arm to which carbon dioxide is tried to be supplied. Next, the both end portions of the envelope body 21 are wrapped toward sides of arrow mark directions. Thereafter, as illustrated in Fig. 2B, when the locking portions 24 provided to the one end portion of the envelope member 22 are locked with respect to the locking portions 24 provided to the other end portion of the envelope member 22, it is possible to attach the envelope body 21 to an arbitrary region of the body in a state where the supply space is sealed. Here, the locking portion 24 uses a face fastener with which locking can be made at an arbitrary position. Accordingly, since it is possible to adjust the positions of locking of the locking portions 24 in accordance with a size of region of the body, one envelope body 21 can be used for different sizes. Further, the locking portions 24 of the present embodiment are provided, in plural numbers, along a width direction of the envelope member 22 (direction of arrow marks illustrated in Fig. 2B). Therefore, even when a region of the body is one whose size is different along the width direction of the envelope member 22 (an arm, a leg, or the like), by adjusting the positions of the respective locking portions 24, it is possible to make the envelope body 21 fit to the region of the body.

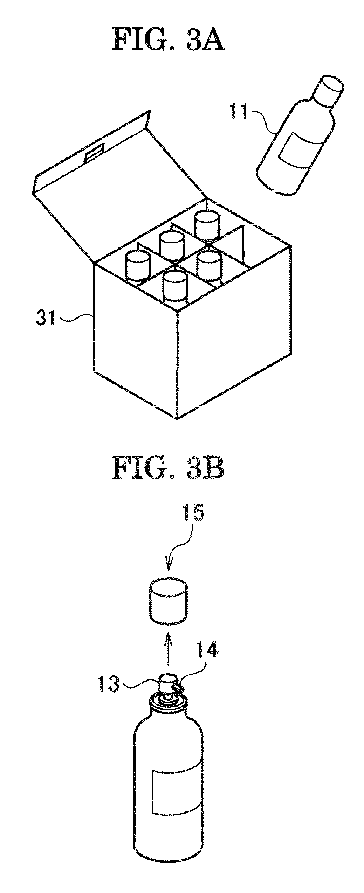

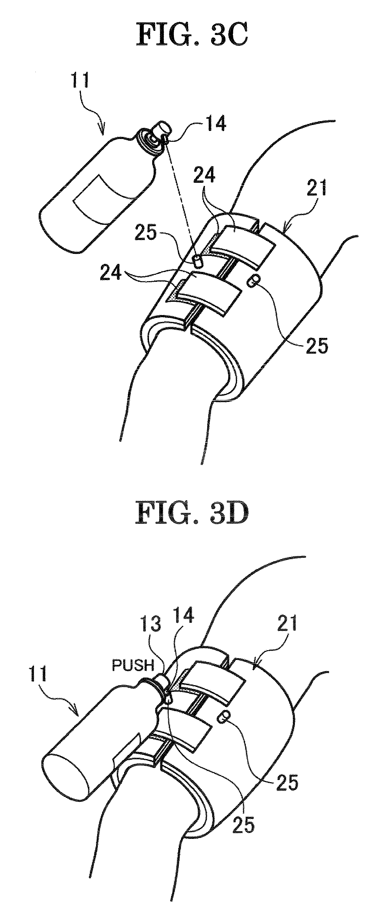

Next, explanation will be made on a method of supplying carbon dioxide with the supply unit, into the envelope body 21 attached to the arm, with reference to Fig. 3A to Fig. 3D. As described above, the supply unit 11 has light weight and is about a size to be portable. Therefore, as illustrated in Fig. 3A, it is possible to make a packing body 31 accommodate a plurality of (six, for example) supply units 11, to manage and carry the supply units. The user takes out the supply unit 11 accommodated in the packing body 31, and, as illustrated in Fig. 3B, removes a cap body 15 that covers the push-down portion 13 and the blow-out port 14 of the supply unit 11. Next, as illustrated in Fig. 3C, the user connects the blow-out port 14 of the supply unit 11 to the suction port 25 of the envelope body 21 attached to the arm. The user pushes, in a state where the suction port 25 and the blow-out port 14 are connected, the push-down portion 13 of the supply unit 11, as illustrated in Fig. 3D. Then, the carbon dioxide and the absorbent material accommodated in the container body 12 are mixed to be discharged into the supply space. At this time, the contact portion 23 of the envelope body 21 is closely contacted with the body surface, so that the carbon dioxide and the absorbent material supplied into the supply space are prevented from being leaked to the outside of the envelope body 21.

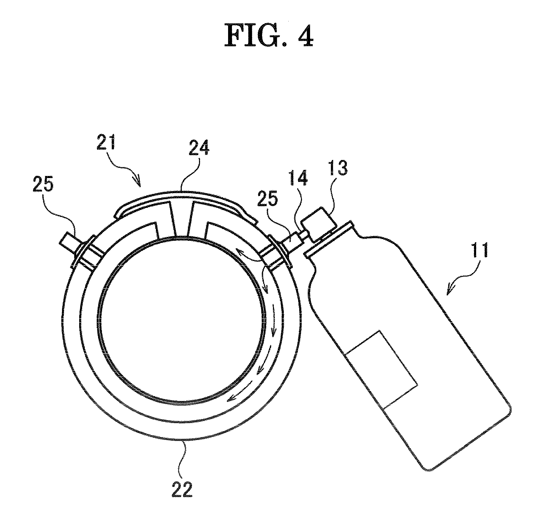

Fig. 4 is a diagram illustrating a cross section of the envelope body 21 in a state where the suction port 25 and the blow-out port 14 illustrated in Fig. 3D are connected, and the push-down portion 13 of the supply unit 11 is pushed. As illustrated in Fig. 4, the carbon dioxide and the absorbent material supplied from the suction port 25 of the envelope body 21 are uniformly filled in the supply space formed around the arm. At this time, the filled absorbent material becomes in a state of being adhered to a surface of the arm by being brought into contact with the arm. The user further keeps pushing the push-down portion 13 of the supply unit 11, from the state illustrated in Fig. 4. Then, although the carbon dioxide and the absorbent material are supplied to the supply space, since the supply space is sealed, the supplied carbon dioxide and absorbent material compress the carbon dioxide filled in the supply space. Note that the compressed carbon dioxide expands the envelope body 21, as illustrated in Fig. 3D. At this time, it is set that an air pressure in the expanded supply space is more than 1 atmosphere and less than 1.3 atmospheres (preferably, not less than 1.05 atmospheres nor more than 1.1 atmospheres). Note that when a pressure equal to or more than a predetermined air pressure is applied, the supplied carbon dioxide is discharged between the contact portion 23 of the envelope body 21 and the body surface.

As described above, the carbon dioxide filled in the supply space is compressed by the further supplied carbon dioxide. A large amount of the pressurized carbon dioxide efficiently dissolves in the absorbent material adhered to the arm. Through the absorbent material in which a large amount of carbon dioxide dissolves, it is possible to make a larger amount of carbon dioxide to be absorbed from the surface of the arm. Further, the absorbent material adhered to the surface of the arm is pressed against the surface of the arm by the carbon dioxide pressurized in the supply space. Therefore, the pressing force realizes a close contact between the absorbent material and the surface of the arm, resulting in that the efficiency for making the carbon dioxide to be absorbed from the surface of the arm can be further improved.

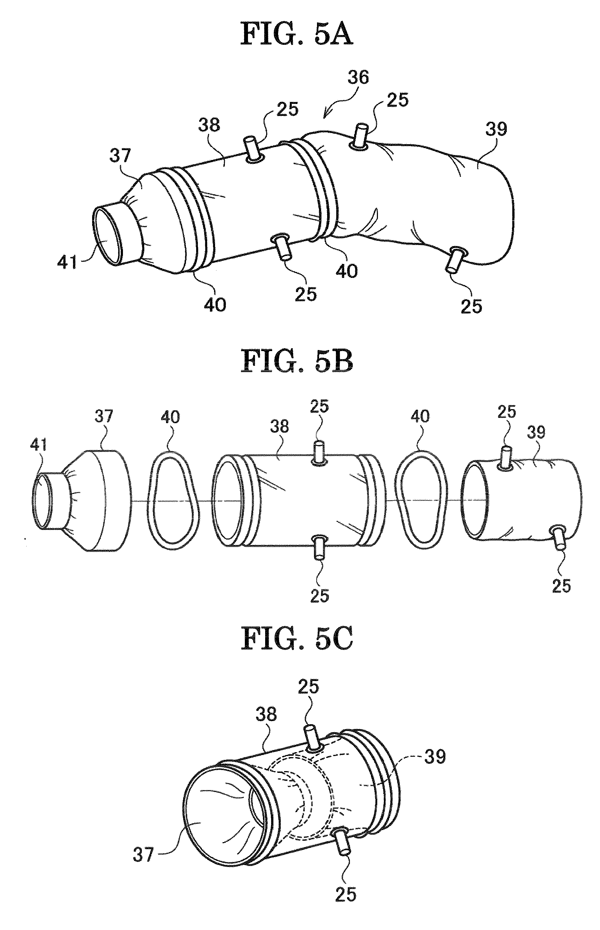

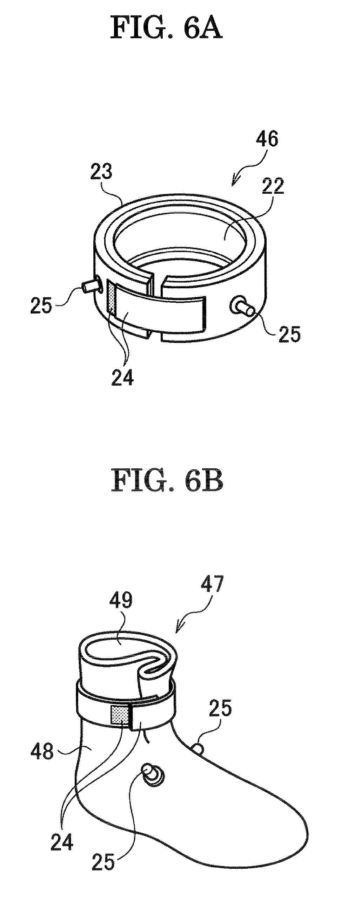

Next, another form of the envelope body used in the present embodiment will be explained with reference to Fig. 5A to Fig. 5C and Fig. 6A to Fig. 6C.
An envelope body 36 illustrated in Fig. 5A is one for arm including an upper arm, a lower arm and a hand. The envelope body 36 has a bag shape, and a supply space to which carbon dioxide is supplied is formed in the envelope body 36. As illustrated in Fig. 5B, the envelope body 36 is formed of a plurality of (three) envelope members 37, 38, 39. As a material of the envelope members 37, 39, there are used a material having elasticity, adhesiveness, flexibility, durability and sealing property, such as, for example, a chloroprene rubber and a material in which a nylon jersey is bonded to the chloroprene rubber (referred to as chloroprene rubber and the like, hereinafter). Meanwhile, the envelope member 38 is formed by using a transparent acrylic as a casing, for example. The envelope member 37 has a cylindrical shape formed so that an opening area thereof is enlarged in stages. To the envelope member 37, there is formed an insertion opening 41 from which an arm can be inserted. The insertion opening 41 is formed to have a size narrower than a size of the upper arm. Therefore, when the user inserts the arm into the envelope body 36, he/she inserts the arm so as to enlarge the insertion opening 41. The envelope member 38 has a cylindrical shape formed to have the same opening size. The envelope member 38 is provided with a plurality of (two) suction ports 25. The envelope member 39 has a bag shape in which only one side is opened. The envelope member 39 is provided with a plurality of (two) suction ports 25, similar to the envelope member 38. The envelope body 36 is structured to have a bag shape, as a whole, by attachably/detachably connecting mutual envelope members adjacent to one another using O rings 40. As above, by attachably/detachably connecting the plurality of envelope members, even when one envelope member is stained or damaged, it is possible to remove the member to clean it or to easily replace the member with a new envelope member.

A method of supplying carbon dioxide to the envelope body 36 is similar to that of the case illustrated in Fig. 3A to Fig. 3D. First, the user connects, in a state where his/her arm is inserted from the insertion opening 41, the blow-out ports 14 of the supply units 11 to the suction ports 25 of the envelope members 38, 39, and pushes the push-down portions 13 of the supply units 11. Then, the carbon dioxide and the absorbent material accommodated in the container body 12 are mixed to be discharged into the supply space. Further, when the push-down portions 13 of the supply units 11 are kept pushed, the carbon dioxide supplied to the supply space is compressed. At this time, a surface of arm of the upper arm and the insertion opening 41 are closely contacted with each other, so that the carbon dioxide and the absorbent material supplied into the supply space are prevented from being leaked to the outside of the envelope body 36.

The envelope body 36 illustrated in Fig. 5A forms a part thereof by using the transparent material (acrylic, for example). By providing a part of the envelope body in a transparent state as described above, the user can visually recognize, through the transparent part, the Bohr effect generated when carbon dioxide is absorbed into the body, for example, a state where a blood circulation is increased and thus a skin becomes red. Further, the envelope body 36 is provided with the plurality of (four) suction ports 25. Accordingly, even if the envelope body 36 becomes large in size, it is possible to make the absorbent material to be uniformly filled in the supply space. Further, the envelope body 36 forms a part thereof as a cylindrical casing. With the structure as described above, it is possible to accommodate deformed envelope members 37, 38 inside the envelope member 38, as illustrated in Fig. 5C, and accordingly, the user can easily manage and carry the envelope body 36.

An envelope body 46 illustrated in Fig. a is one for neck. The envelope body 46 is formed similarly to the envelope body 21 illustrated in Fig. 1. The envelope body 46 illustrated in Fig. 6A is different from the envelope body 21 illustrated in Fig. 1 in that only one pair of locking portions 24 are provided. Note that a length dimension (circumferential length) of the envelope member 22 of the envelope body 46 for neck illustrated in Fig. 6A is also possible to be structured to have a length dimension shorter than a neck size in order not to envelop a region of throat of the neck enveloped by the envelope body 46.

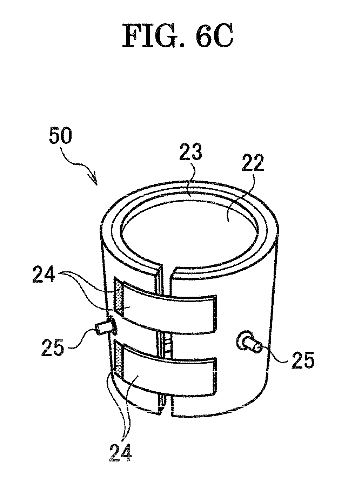

An envelope body 47 illustrated in Fig. 6B is one for foot. The envelope body 47 has a bag shape formed in a shape of foot, and a supply space to which carbon dioxide is supplied is formed in the envelope body 47. As a material of an envelope member 48 of the envelope body 47, a chloroprene rubber and the like are used, for example. To the envelope member 48, there is formed an insertion opening 49 from which a foot can be inserted. When the user attaches the envelope body 47, he/she inserts his/her foot from the insertion opening 49, and then locks the locking portion 24 provided at a position corresponding to a region of ankle of the envelope body 47 with respect to the other locking portion 24, and accordingly, the envelope body 47 can be attached while sealing the supply space.
An envelope body 50 illustrated in Fig. 6C is one for femur. The envelope body 50 is formed similarly to the envelope body 21 illustrated in Fig. 1. Note that by slightly shortening a length dimension (circumferential length) of the envelope member 22 of the envelope body 50, the envelope body can be used for crus.

As described above, according to the present embodiment, the carbon dioxide filled in the supply space is pressurized by the discharge pressure when discharging the carbon dioxide with the use of the supply unit. Therefore, it is possible to make a large amount of carbon dioxide to be dissolved in the absorbent material, so that a large amount of carbon dioxide can be absorbed into the body through the absorbent material in which a large amount carbon dioxide is dissolved, and further, it is possible to improve the efficiency for making the carbon dioxide to be absorbed into the body. Further, since the absorbent material is pressed against the body surface by the pressurized carbon dioxide, the absorbent material and the surface of the arm are closely contacted with each other, resulting in that the efficiency for making the carbon dioxide to be absorbed into the body can be further improved.

Further, in the present embodiment, the supply unit accommodates the carbon dioxide to be supplied into the body and the absorbent material through which the carbon dioxide is absorbed into the body from the body surface, in a highly compressed state. Therefore, since the user can supply the absorbent material together with the carbon dioxide to the supply space, only by supplying the carbon dioxide using the supply unit, there is no need to put on or apply the absorbent material, and it is possible to improve usability when supplying the carbon dioxide.

Further, in the present embodiment, the material of the envelope body 21 employs the chloroprene rubber, for example. The chloroprene rubber has the elasticity as well as the adhesiveness. Therefore, the chloroprene rubber can be closely contacted with any region of the body, and it is also possible to prevent the filled carbon dioxide from being leaked. For example, even when the chloroprene rubber is applied to an uneven region such as elbow, it expands and contracts along a shape of the elbow to be closely contacted with the elbow. Further, by bonding the nylon jersey to the chloroprene rubber, the durability is further enhanced, so that even when pressurization is made by the carbon dioxide filled in the supply space, it is possible to prevent the damage of the envelope body. Note that the envelope body 21 may also use a cloth with water repellency.

### (Second Embodiment)

A second embodiment is an embodiment of a case where a supply unit supplying carbon dioxide into an envelope body enveloping at least a part of body surface and a pressuring unit pressurizing the carbon dioxide supplied into the envelope body to make the carbon dioxide to be absorbed into an absorbent material, are made common. In the first embodiment, the carbon dioxide and the absorbent material are discharged from the supply unit to make the absorbent material adhere to the surface of the body, but, in the second embodiment, a method of supplying the absorbent material is different.

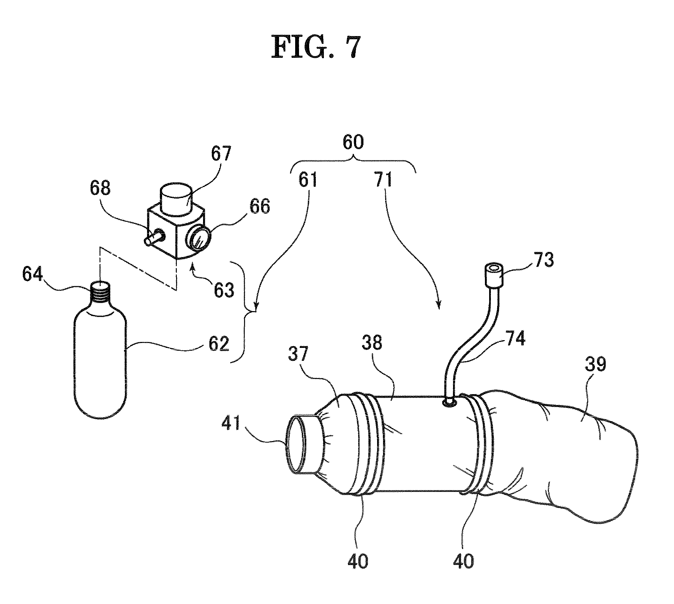

Fig. 7 illustrates a structure of a carbon dioxide supply device 60 according to the second embodiment. As illustrated in Fig. 7, the carbon dioxide supply device 60 includes a supply unit 61 and an envelope body 71.

The supply unit 61 includes a container body 62 and a pressure reducer 63. The container body 62 is a so-called handy cylinder, in which carbon dioxide is accommodated in a very highly compressed state. In the container body 62 of the present embodiment, carbon dioxide of about 10 to 20 liters is accommodated. The pressure reducer 63 includes a scale portion 66, a knob portion 67, and a blow-out port 68. When the pressure reducer 63 is attached to the container body 62 via a screw portion 64 of the container body 62, it is possible to take out the carbon dioxide highly compressed in the container body 62 by reducing the pressure of carbon dioxide. When a user opens the knob portion 67, the carbon dioxide accommodated in the container body 62 can be vigorously discharged from the blow-out port 68. Note that the user can check the pressure of the carbon dioxide discharged from the blow-out port 68, with the use of the scale portion 66.

The envelope body 71 is one for arm including an upper arm, a lower arm and a hand, being at least a part of body surface. The envelope body 71 has a bag shape, and a supply space to which carbon dioxide is supplied is formed in the envelope body 71. Here, the envelope body 71 is formed similarly to the envelope body 36 illustrated in Fig. 5A. The envelope body 71 illustrated in the present embodiment is different from the envelope body 36 illustrated in Fig. 5A in that a suction port 73 is provided via a suction tube 74. To the suction port 73, the blow-out port 68 of the supply unit 61 can be connected. In the supply unit 61 according to the present embodiment, the carbon dioxide is accommodated in a very highly compressed state, so that the supply unit 61 is heavier than the supply unit 11 of the first embodiment. By providing the suction tube 74, the user does not have to hold the supply unit 61 in his/her hand, and it is possible to supply carbon dioxide into the envelope body 71 from the supply unit 61 in a state of being placed.

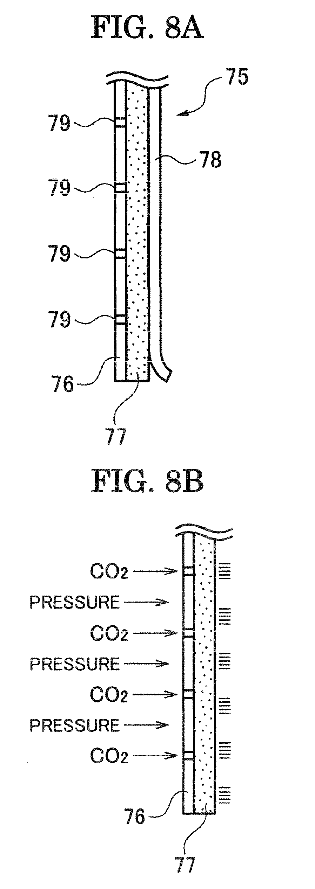

Next, a method of supplying an absorbent material according to the present embodiment will be described. As the method of supplying the absorbent material, firstly, there is a method of putting a sheet-shaped absorbent material on a surface of a body. Here, explanation will be made on the sheet-shaped absorbent material with reference to Fig. 8A, Fig. 8B. An absorbent material 75 is previously formed or cut in a size corresponding to a size of a region of the body into which carbon dioxide is tried to be absorbed. As illustrated in Fig. 8A, the absorbent material 75 is formed of layers with a plurality of different materials, and includes a permeable sheet 76, an absorbent part 77, and a laminate sheet 78. On the permeable sheet 76, there are formed a plurality of holes 79 through which the carbon dioxide can permeate. The absorbent part 77 is a part to be put on the surface of the body. The absorbent part 77 is a medium in which carbon dioxide can be dissolved, and is water, alcohols, oils and fats or the like, for example. Further, the absorbent part 77 is preferably gel-type one so that when it is put on the surface of the body, the put absorbent material is easily remained at the put position. For example, as the gel-type absorbent part 77, a high molecular compound (sodium polyacrylate) may be added to form gel. Further, the absorbent part 77 is slightly acidic with PH of 4.0 to 6.5 for reducing an influence on the body surface, and glycerin or Vaseline is added thereto for moisturizing and protecting the body surface. The laminate sheet 78 is for protecting the absorbent part 77 from dust and the like. When the user uses the absorbent material 75, he/she peels off the laminate sheet 78 from the absorbent part 77. As above, by forming the absorbent material 75 in a sheet shape, the absorbent material is easily managed and easily portable. Note that as the absorbent part, it is also possible to use a substance such as a cloth in which a medium such as water, alcohols, oils and fats or the like in which carbon dioxide can be dissolved is permeated. Further, the medium such as water, alcohols, oils and fats or the like is preferably nano-sized one so that it is easily absorbed into the body from the body surface.

As the method of supplying the absorbent material, secondly, there is a method of directly applying the absorbent material to the surface of the body. Here, the absorbent material has a similar physical property to that of the absorbent part 77 described in the first method. Note that it is preferable that the absorbent material applied to the surface of the body is further remained at the applied position by setting such that the absorbent material itself has a viscosity, or the absorbent material is made to contain a viscosity agent.

Next, a method of supplying carbon dioxide using the supply unit into the envelope body 71 attached to the arm will be described. Here, the method of supplying the absorbent material is assumed to be conducted using the aforementioned first method in which the sheet-shaped absorbent material is put on the surface of the body. First, the user peels off the laminate sheet 78 of the sheet-shaped absorbent material 75, and then puts the absorbent part 77 of the absorbent material 75 on the body surface of an upper arm, a lower arm, a back of a hand and the like. Next, the user takes out the container body 62 of the supply unit 61 accommodated in a packing body, for example, and attaches the pressure reducer 63 to the container body 62 via the screw portion 64. Subsequently, the user connects the blow-out port 68 of the pressure reducer 63 and the suction port 73 at a tip of the suction tube 74 of the envelope body 71.

Further, the user inserts his/her arm from the insertion opening 41 of the envelope body 71, and then opens the knob portion 67 of the pressure reducer 63. Then, the carbon dioxide accommodated in the container body 62 is discharged into the supply space of the envelope body 71. When the user keeps opening the knob portion 67, the carbon dioxide filled in the supply space is compressed by the carbon dioxide which is continuously supplied. A large amount of the pressurized carbon dioxide efficiently dissolves in the absorbent part 77 put on the arm by permeating through the holes of the permeable sheet 76, as illustrated in Fig. 8B. Through the absorbent part 77 in which a large amount of carbon dioxide dissolves, it is possible to make a larger amount of carbon dioxide to be absorbed from the surface of the arm. Further, the absorbent part 77 put on the surface of the arm is pressed against the side of the surface of the arm via the permeable sheet 76 by the carbon dioxide pressurized in the supply space. Therefore, the pressing force realizes a close contact between the absorbent part 77 and the surface of the arm, resulting in that the efficiency for making the carbon dioxide to be absorbed from the surface of the arm can be further improved.

Note that the above method can be conducted in a similar manner even in a case where the method of supplying the absorbent material employs the aforementioned second method in which the absorbent material is applied to the surface of the body. Further, when putting on the sheet-shaped absorbent material 75, to a region of body such as elbow on which the sheet-shaped absorbent material 75 is difficult to be put, the gel-type absorbent material may by applied, and thus it is also possible to use the sheet-shaped absorbent material 75 and the gel-type absorbent material in a combined manner. Further, in addition to the cases of putting on the sheet-shaped absorbent material 75 and applying the gel-type absorbent material, it is also possible to design such that the carbon dioxide and the absorbent material are discharged simultaneously from the supply unit 61. For example, in this case, a spray port of a sprayer capable of spraying the absorbent material in a mist form may be provided in proximity to the blow-out port 68 of the pressure reducer 63.

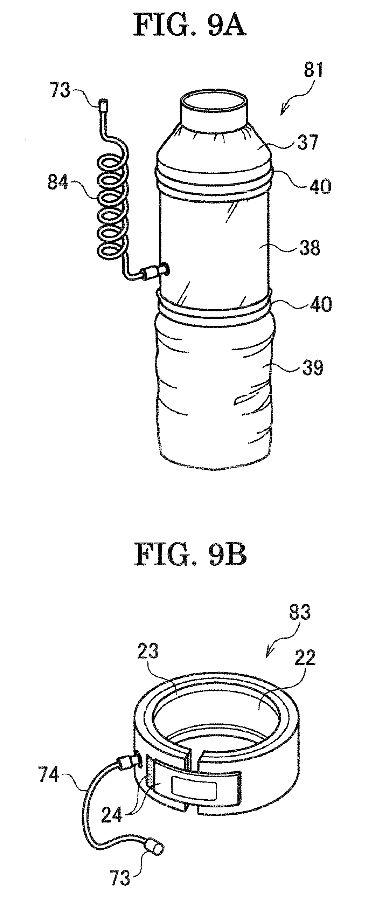

Next, another form of the envelope body used in the present embodiment will be described with reference to Fig. 9A to Fig. 9D.
An envelope body 81 illustrated in Fig. 9A is one for leg including a femur, a crus and a foot. The envelope body 81 is formed similarly to the envelope body 71 illustrated in Fig. 7. The envelope body 81 illustrated in Fig. 9A is different from the envelope body 71 illustrated in Fig. 7 in that it is formed to be large in size for leg.
An envelope body 83 illustrated in Fig. 9B is one for neck. The envelope body 83 is formed similarly to the envelope body 46 illustrated in Fig. 6A. The envelope body 83 illustrated in Fig. 9B is different from the envelope body 46 illustrated in Fig. 6A in that there is provided a suction port 73 via a suction tube 74.

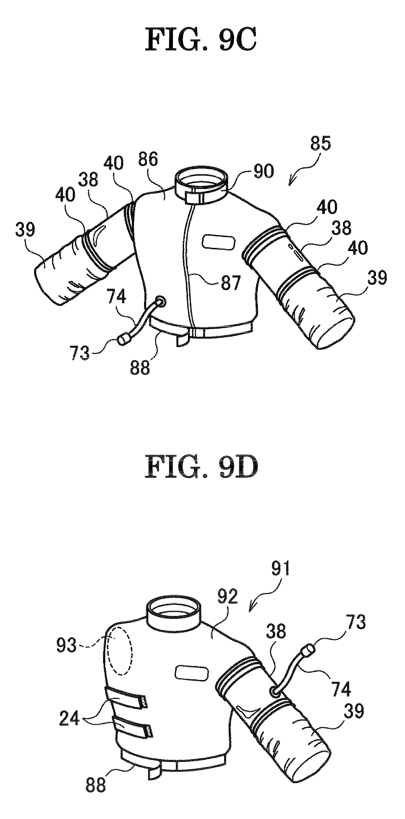

An envelope body 85 illustrated in Fig. 9C is one for upper half of body including both arms. The envelope body 85 is formed so that it can envelop, among regions of body, a region above a lumbar and below a neck. The envelope body 85 is formed of a plurality of (five) envelope members 38, 39, 86. The envelope members 38, 39 are formed similarly to the envelope members 38, 39 illustrated in Fig. 7. As a material of the envelope member 86, a chloroprene rubber is used, for example. Further, the envelope member 86 has a so-called vest shape, and includes an airtight fastener 87, an abdomen locking portion 88, a neck locking portion 90, a suction tube 74 and a suction port 73. When a user attaches the envelope body 85, he/she opens the airtight fastener 87, and then attaches the envelope body 85 like putting on clothes. Next, the user closes the airtight fastener 87 and performs locking by using the abdomen locking portion 88 and the neck locking portion 90, resulting in that a sealed supply space is formed around the upper half of body including the both arms. Note that the envelope body 85 is formed in a rather large size in order not to tighten the upper half of body except for the regions of neck and abdomen. Further, since the envelope body 85 employs the material with elasticity such as the chloroprene rubber for the envelope member 39 of a portion corresponding to the hand, the user can easily perform the work of opening/closing the airtight fastener 87 and the like.

An envelope body 91 illustrated in Fig. 9D is one for upper half of body (for shoulder) including one arm. The envelope body 91 is formed by eliminating the envelope members 38, 39 of one arm region of the envelope body 85 illustrated in Fig. 9C. The envelope body 91 is formed of a plurality of (three) envelope members 38, 39, 92. The envelope body 91 includes locking portions 24 and an abdomen locking portion 88, and it also includes an arm opening 93 from which the other arm that is not enveloped by the envelope members 38, 39 is exposed to the outside of the envelope body 91. When a user attaches the envelope body 91, he/she attaches it like putting on clothes. Next, the user performs locking by using the locking portions 24 and the abdomen locking portion 88, resulting in that a sealed supply space is formed around the upper half of body including the one arm.

As described above, according to the present embodiment, there is used a small-sized supply unit (handy-type cylinder) capable of accommodating carbon dioxide to be supplied into the body, in a very highly compressed state. Therefore, since it is possible to discharge a large amount of carbon dioxide, the carbon dioxide can be supplied to a wide range of the surface of the body.
Further, in the present embodiment, as the method of supplying the absorbent material, the method of putting on the sheet-shaped absorbent material and the method of applying the gel-type absorbent material are used. Accordingly, the absorbent material can be securely adhered to the surface of the body, so that the efficiency for making the carbon dioxide to be absorbed into the body can be improved.

### (Third Embodiment)

A third embodiment is an embodiment of a case where a supply unit supplying carbon dioxide into an envelope body enveloping at least a part of body surface and a pressuring unit pressurizing the carbon dioxide supplied into the envelope body to make the carbon dioxide to be absorbed into an absorbent material, are separately provided.

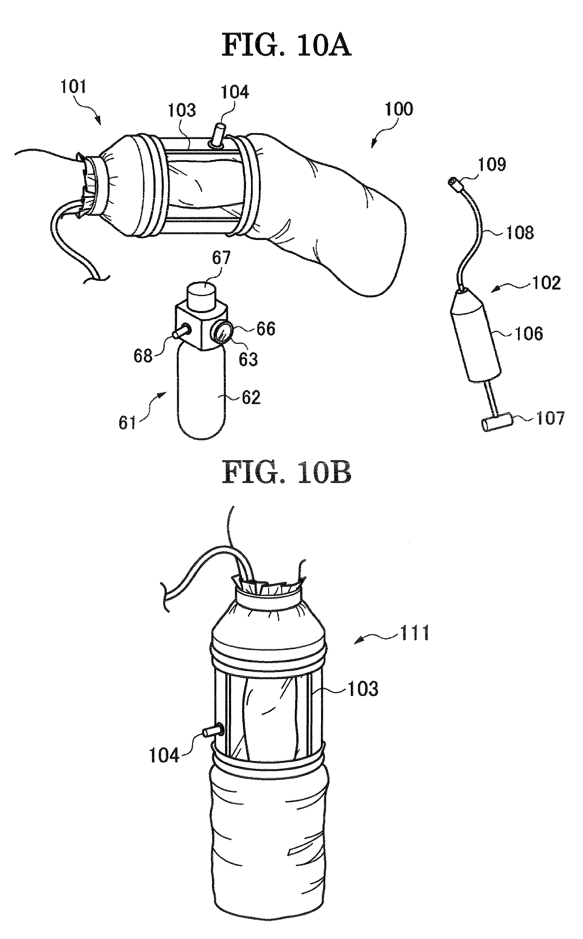

Fig. 10A, Fig. 10B illustrate a structure of a carbon dioxide supply device 100 according to the third embodiment. As illustrated in Fig. 10A, the carbon dioxide supply device 100 includes a supply unit 61, an envelope body 101 and a pressurizing unit 102.

The supply unit 61 includes a container body 62 and a pressure reducer 63. The container body 62 is a so-called handy cylinder, in which carbon dioxide is accommodated in a very highly compressed state. When a user opens a knob portion 67, the carbon dioxide accommodated in the container body 62 can be vigorously discharged from a blow-out port 68.
The envelope body 101 is one for arm including an upper arm, a lower arm and a hand being at least a part of the body surface. The envelope body 101 has a bag shape, and a supply space to which carbon dioxide is supplied is formed in the envelope body 101. Further, there is provided, inside the envelope body 101, a partition member 103 that partitions the supply space into the body surface side and the envelope body side. The partition member 103 illustrated in Fig. 10A is formed to have a bag shape so as to cover the arm inside the envelope body 101. For the partition member 103, a material having impermeability to gas, and elasticity such as, for example, a vinyl being a polymeric resin is used. Note that the partition member 103 may also be integrally provided in the envelope body 101. On an outer periphery of the envelope body 101, there is provided a suction port 104 from which compressed substance of gas or the like is sucked, in a space within the supply space and between the partition member 103 and the envelope body 101.
The pressurizing unit 102 includes a cylindrical main body 106, an operation part 107, a delivery tube 108 and a delivery port 109. The pressurizing unit 102 is a so-called handy-type pump. When a user makes the operation part 107 move forward and backward with respect to the cylindrical main body 106, it is possible to discharge gas in the cylindrical main body 106 from the delivery port 109.

Next, explanation will be made on a method in which the user attaches the envelope body 101. First, the user applies the gel-type absorbent material to a surface of the entire arm of the upper arm, the lower arm, the hand and the like. Next, the user covers the entire arm of the upper arm, the lower arm, the hand and the like so as to put the bag-shaped partition member 103 on the entire arm. Further, the user inserts the entire arm covered by the partition member 103 from an insertion opening of the envelope body 101. At this time, by providing a state in which an opening portion of the partition member 103 is exposed from the insertion opening of the envelope body 101 as illustrated in Fig. 10A, gas filled in a space between the partition member 103 and the envelope body 101 and gas filled in a space between the body surface and the partition member 103 can be prevented from being mixed.

Next, the user connects the suction tube to the blow-out port 68 of the supply unit 61, inserts a tip of the suction tube into the bag-shaped partition member 103, namely, a space surrounded by the body surface and the partition member 103, and then opens the knob portion 67 of the pressure reducer 63. Then, the carbon dioxide accommodated in the container body 62 is discharged into the bag-shaped partition member 103 of the envelope body 101, namely, the space surrounded by the body surface and the partition member 103. Here, the user closes the knob portion 67 of the pressure reducer 63 when the carbon dioxide is filled in the partition member 103 to some degree. At this state, the carbon dioxide in the partition member 103 is not yet pressurized.

Next, the user connects the delivery port 109 of the pressurizing unit 102 to the suction port 104 of the envelope body 101, and makes the operation part 107 move forward and backward with respect to the cylindrical main body 106. Then, the gas in the cylindrical main body 106 can be discharged to the space between the partition member 103 and the envelope body 101. When the user keeps supplying the gas to the space between the partition member 103 and the envelope body 101, the gas filled in the space between the partition member 103 and the envelope body 101 compresses the carbon dioxide in the partition member 103 via the partition member 103. Note that the gas filled in the space between the partition member 103 and the envelope body 101 expands the envelope body 101. At this time, it is set that an air pressure in the expanded supply space is more than 1 atmosphere and less than 1.3 atmospheres (preferably, not less than 1.05 atmospheres nor more than 1.1 atmospheres). A large amount of the pressurized carbon dioxide efficiently dissolves in the absorbent material applied to the arm. Through the absorbent material in which a large amount of carbon dioxide dissolves, it is possible to make a larger amount of carbon dioxide to be absorbed from the surface of the arm. Further, the absorbent material adhered to the surface of the arm is pressed against the surface of the arm by the carbon dioxide pressurized in the supply space. Therefore, the pressing force realizes a close contact between the absorbent material and the surface of the arm, resulting in that the efficiency for making the carbon dioxide to be absorbed from the surface of the arm can be further improved.

Note that in the aforementioned embodiment, the pressurizing unit 102 uses the handy-type pump, but, the present invention is not limited to this case. For example, it is also possible to design such that a compression machine such as a compressor or a pressure machine which is referred to a so-called MEDOMER (registered trademark) is used, to thereby supply the gas into the envelope body 101. Further, it is also possible to design such that, for example, a bellows unit having a space therein is used, and the gas is supplied into the envelope body by expanding and contracting a bellows portion. Further, what is supplied into the envelope body 101 is not limited to the gas. It may also be liquid or solid substance, for example. When supplying the solid substance, by structuring such that the solid substance directly presses the outside of the partition member, it is possible to pressurize the carbon dioxide. Further, when supplying the solid substance, it is possible to structure such that the solid substance directly presses the outside of the envelope body according to the first embodiment and the second embodiment.
Further, in the aforementioned embodiment, a case where the gel-type absorbent material is applied to the surface of the entire arm of the upper arm, the lower arm, the hand and the like, is described, but, the present invention is not limited to this case, and it is also possible to put the sheet-shaped absorbent material on the surface of the upper arm, the lower arm, the hand and the like.

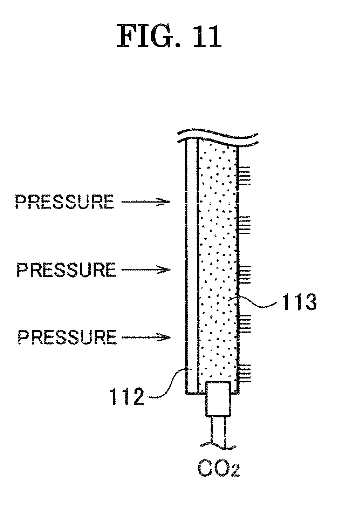

Further, as the partition member, the bag-shaped one is used, but, the present invention is not limited to this case. For example, it is also possible that the partition member is formed in a sheet shape and is put on the body surface. Here, the sheet-shaped partition member will be described with reference to Fig. 11. A partition member 112 uses a material having impermeability to gas. To the partition member 112, an absorbent part 113 to be put on the surface of the body is adhered. The absorbent part 113 is a substance such as a sponge in which a medium in which carbon dioxide can be dissolved is permeated. Note that the medium is water, alcohols, oils and fats or the like, and structured to have a similar physical property to that of the second embodiment. Next, explanation will be made on a method in which a user uses the partition member 112 to supply carbon dioxide into the envelope body 101. First, the user puts the partition member 112 on the body surface so that the absorbent part 113 faces the body surface side. Next, the user inserts the entire arm on which the partition member 112 is put from the insertion opening of the envelope body 101. The user connects the suction tube to the blow-out port 68 of the supply unit 61, and inserts a tip of the suction tube into the absorbent part 113 as illustrated in Fig. 11, thereby discharging carbon dioxide into the absorbent part 113, namely, a space in the absorbent part 113 surrounded by the body surface and the partition member 103. Next, when the user supplies gas to a space between the partition member 112 and the envelope body 101 using the pressurizing unit 102, the gas filled in the space between the partition member 112 and the envelope body 101 compresses the carbon dioxide in the absorbent part 113 via the partition member 112, resulting in that a large amount of carbon dioxide efficiently dissolves in the medium. Through the medium in which a large amount of carbon dioxide dissolves, it is possible to make a larger amount of carbon dioxide to be absorbed from the surface of the arm.

Next, another form of the envelope body used in the present embodiment will be described with reference to Fig. 10B. An envelope body 111 illustrated in Fig. 10B is one for leg including a femur, a crus and a foot. The envelope body 111 is formed similarly to the envelope body 101 illustrated in Fig. 10A. Further, similarly, a partition member 103 that partitions a supply space into the body surface side and the envelope body side is also provided inside the envelope body 111. The envelope body 111 illustrated in Fig. 10B is different from the envelope body 101 illustrated in Fig. 10A in that it is formed to be large in size for leg.

As described above, according to the present embodiment, there is provided the partition member, inside the envelope body, that partitions the supply space into the body surface side and the envelope body side. Further, it is designed such that the carbon dioxide is supplied only to the space surrounded by the body surface and the partition member 103. Therefore, it is possible to reduce the amount of carbon dioxide to be supplied. Further, even when the absorbent material is applied to the body surface, the supply space is partitioned by the partition member into the body surface side and the envelope body side, so that it is possible to prevent the absorbent material from being adhered to the envelope body.

### (Fourth Embodiment)

A fourth embodiment is an embodiment suitable for a case where a large amount of carbon dioxide is supplied into an envelope body enveloping at least a part of body surface.

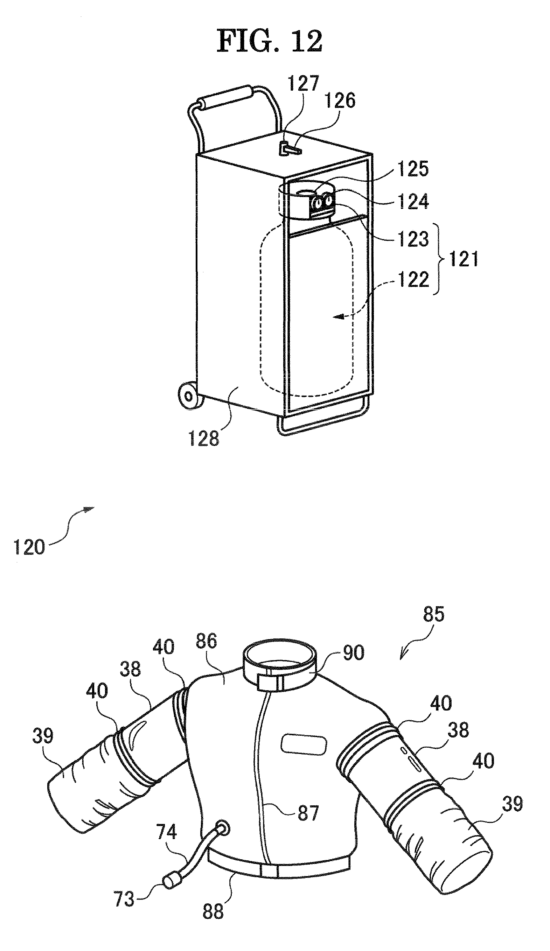

Fig. 12 illustrates a structure of a carbon dioxide supply device 120 according to the fourth embodiment. As illustrated in Fig. 12, the carbon dioxide supply device 120 includes a supply unit 121 and an envelope body 85.

The supply unit 121 includes a container body 122 and a pressure reducer 123. The container body 122 is a so-called large-sized cylinder, in which a large amount of carbon dioxide is accommodated in a very highly compressed state. The pressure reducer 123 includes a scale portion 124 and a stopper portion 125. Note that the supply unit 121 is accommodated in a case body 128. The case body 128 includes a blow-out port 127 and a cock portion 126 opening/closing the blow-out port 127. Further, casters or the like are attached to the case body 128, which enables to easily carry the supply unit 121. Note that the stopper portion 125 of the pressure reducer 123 is connected to the blow-out port 127 via a tube, and when a user opens the stopper portion 125 and the cock portion 126, the carbon dioxide accommodated in the container body 122 is vigorously discharged from the blow-out port 127. Note that the user can check the pressure of the carbon dioxide discharged from the blow-out port 127, with the use of the scale portion 124.

The envelope body 85 is one for upper half of body including both arms. The envelope body 85 is formed so that it can envelop, among regions of body, a region above a lumbar and below a neck. The envelope body 85 illustrated in Fig. 12 has the same structure as that of the envelope body 85 illustrated in Fig. 9C.

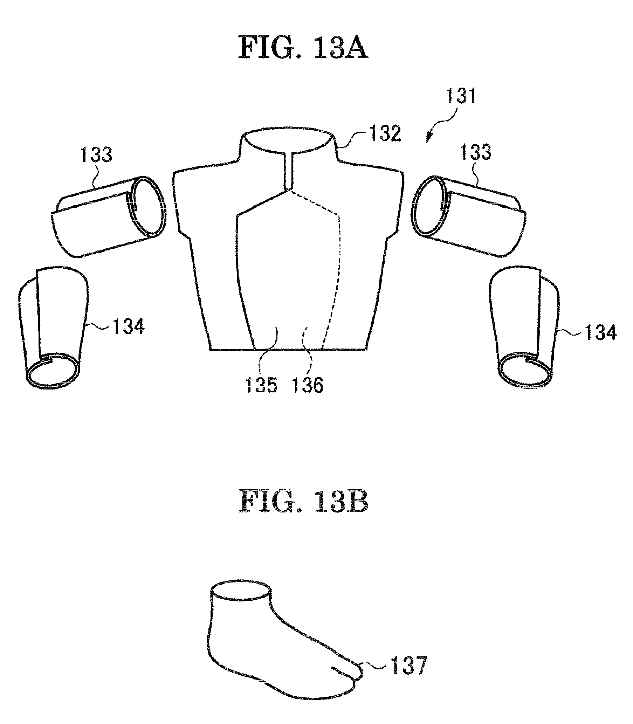

Explanation will be made on a method of supplying an absorbent material according to the present embodiment. As the method of supplying the absorbent material, there are a method of putting a sheet-shaped absorbent material on a surface of a body, and a method of directly applying the absorbent material to the surface of the body. Here, the absorbent material suitable for a case where the body is enveloped by the envelope body 85 for upper half of body will be described with reference to Fig. 13A to Fig. 13C.
In Fig. 13A, an absorbent material 131 is formed of a plurality of (five) absorbent materials 132, 133, 134. Each of the absorbent materials 132, 133, 134 is formed of the same substance as that of the absorbent material illustrated in Fig. 8B. The absorbent material 132 has a so-called vest shape, and by putting it on the upper half of body except for the both arms, it is possible to make an absorbent part of the absorbent material 132 adhere to the body surface. Further, the absorbent material 132 has extra portions 135, 136 overlapping each other from both sides on a front side, so that adjustment can be made in accordance with a size of the body on which the absorbent material 132 is put. By providing the extra portions 135, 136 as above, it is possible to deal with various sizes using one absorbent material 132. When the absorbent materials 133 are put on upper arms of the both arms by being wrapped around the arms, it is possible to make absorbent parts of the absorbent materials 133 adhere to the body surface. Further, when the absorbent materials 134 are put on lower arms of the both arms by being wrapped around the arms, it is possible to make absorbent parts of the absorbent materials 134 adhere to the body surface.

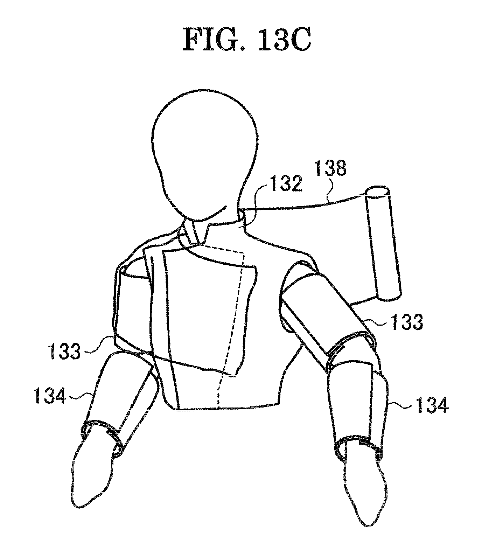

Note that by forming the absorbent material 131 to have a shape in accordance with a shape of region of the body, the attachment of the absorbent material 131 can be easily conducted, and the absorbent part of the absorbent material 131 is likely to be closely contacted with the surface of the body, resulting in that a larger amount of carbon dioxide can be supplied into the body. For example, as illustrated in Fig. 13B, it is also possible to form an absorbent material 137 with a shape in accordance with a foot shape. Note that when putting on the absorbent material 133, to a region of the body such as elbow on which the material is difficult to be put, the absorbent material is applied.
Further, in the present embodiment, after putting the absorbent material 131 on the surface of the body, a permeable film material 138 through which carbon dioxide can permeate is wrapped around the body over the absorbent material 131, as illustrated in Fig. 13C. Here, the permeable film member 138 is, for example, a wrap film on which holes through which carbon dioxide permeates are formed, a fine mesh nonwoven fabric or the like.

Next, explanation will be made on a method of supplying carbon dioxide into the envelope body 85 attached to the upper half of body using the supply unit. Here, the method of supplying the absorbent material is assumed to be conducted by putting the sheet-shaped absorbent material 131 on the surface of the body. First, a user peels off a laminate portion of the sheet-shaped absorbent material 131, and then puts each of the absorbent parts of the absorbent materials 132, 133, 134 on the surface of the upper half of body, the upper arm and the lower arm, respectively. Note that when putting on the absorbent material 131, it is preferable that the user warms the absorbent material 131 to, for example, 30°C to 35°C and the like, so that the body is not cooled. Further, to the elbow or the like on which the absorbent material 131 is difficult to be put, a gel-type absorbent material is applied, for example. Next, after attaching the envelope body 85 illustrated in Fig. 12, the user closes the airtight fastener 87, and performs locking by using the abdomen locking portion 88 and the neck locking portion 90, resulting in that a sealed supply space is formed around the upper half of body including the both arms.

Next, the user connects the suction port 73 of the suction tube 74 to the blow-out port 127 of the case body 128 in which the supply unit 121 is accommodated. The user opens the stopper portion 125 and the cock portion 126. Then, the carbon dioxide accommodated in the container body 122 is discharged into the supply space of the envelope body 85. When the user keeps opening the stopper portion 125 and the cock portion 126, the carbon dioxide filled in the supply space is compressed by the carbon dioxide which is continuously supplied. A large amount of the pressurized carbon dioxide permeates through the permeable film member 138, further permeates through the holes of the permeable sheet 76 of the absorbent material 131, and efficiently dissolves in the absorbent parts put on the surface of the upper half of body including the both arms and the applied absorbent material. Further, the permeable film member 138 wrapped over the sheet-shaped absorbent material 131 and the gel-type absorbent material is pressed against the side of the surface of the upper half of body by the carbon dioxide pressurized in the supply space. Therefore, the pressing force realizes a close contact between the absorbent parts and the surface of the upper half of body including the both arms, resulting in that the efficiency for making the carbon dioxide to be absorbed from the surface of the arms can be further improved. Further, since the permeable film member 138 is wrapped over the entire surface of the upper half of body, the carbon dioxide can be efficiently absorbed from the entire surface of the upper half of body. Further, by wrapping the permeable film member 138 over the entire surface of the upper half of body, it is possible to prevent a stain caused when the absorbent material adheres to the envelope body. Note that the sheet-shaped absorbent material can also be changed to a substance such as a cloth in which a medium such as water, alcohols, oils and fats or the like in which carbon dioxide can be dissolved is permeated. Further, the medium such as water, alcohols, oils and fats or the like is preferably nano-sized one so that it is easily absorbed into the body from the body surface.

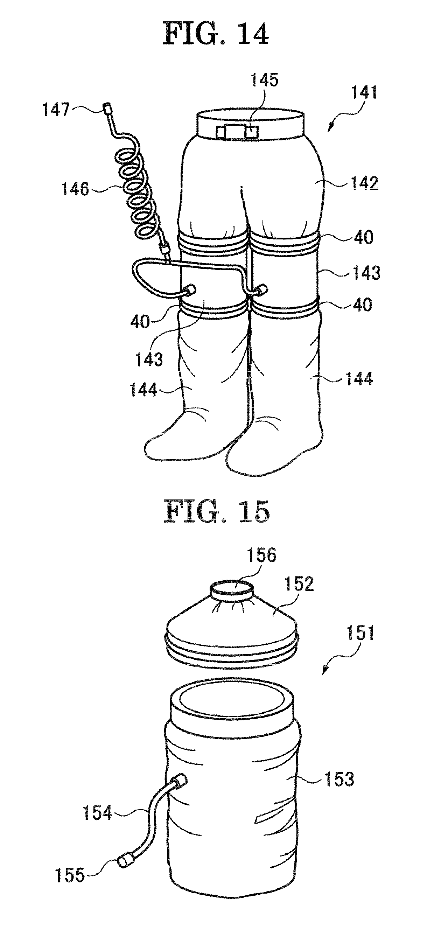

Next, another form of the envelope body used in the present embodiment will be described with reference to Fig. 14 to Fig. 17B.
An envelope body 141 illustrated in Fig. 14 is one for lower half of body including a lumbar and both legs. The envelope body 141 has a bag shape, and a supply space to which carbon dioxide is supplied is formed in the envelope body 141. The envelope body 141 is formed of a plurality of (five) envelope members 142, 143, 144. A material of the envelope members 142, 144 employs a chloroprene rubber and the like, for example. Further, the envelope member 142 uses a transparent acrylic as a casing, for example. The envelope member 142 includes a lumbar locking portion 145. By locking the lumbar locking portion 145, it is possible to prevent the carbon dioxide supplied into the supply space from being leaked to the outside. The envelope members 143 include a suction port 147 via a suction tube 146.

An envelope body 151 illustrated in Fig. 15 is one for whole body except for a head. The envelope body 151 has a bag shape, and a supply space to which carbon dioxide is supplied is formed in the envelope body 151. The envelope body 151 is formed of a plurality of (two) envelope members 152, 153. A material of the envelope members 152, 153 employs a chloroprene rubber and the like, for example. The envelope member 152 includes an opening portion 156 from which the head can be exposed to the outside of the envelope body 151. Further, the envelope member 153 includes a suction port 155 via a suction tube 154.

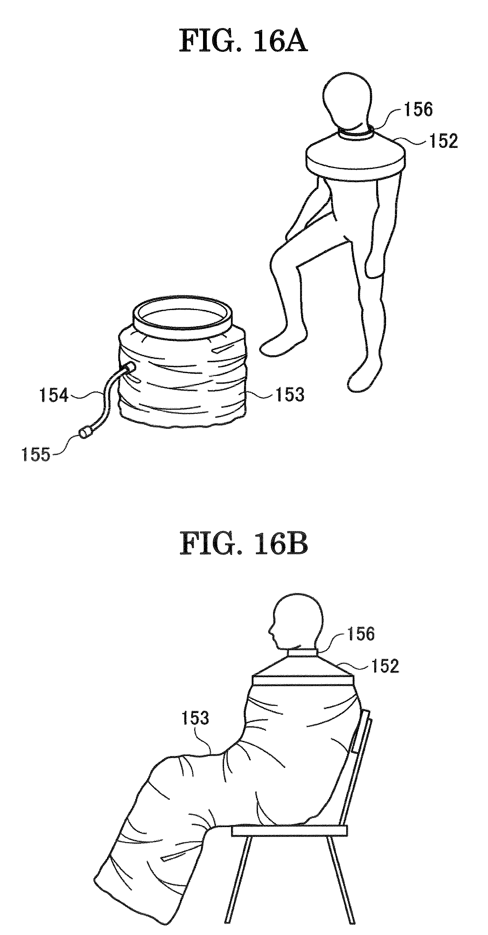

Next, explanation will be made on an attachment method of the envelope body 151 with reference to Fig. 16A, Fig. 16B. First, a user exposes his/her head to the outside from the opening portion 156 of the envelope member 152 so as to pull the envelope member 152 over his/her head, as illustrated in Fig. 16A. Next, the user inserts the both legs into a space in the envelope member 153, and connects the envelope member 152 and the envelope member 153. Fig. 16B is a diagram illustrating a state where the envelope member 152 and the envelope member 153 are connected, and the whole body except for the head is enveloped by the envelope body 151.

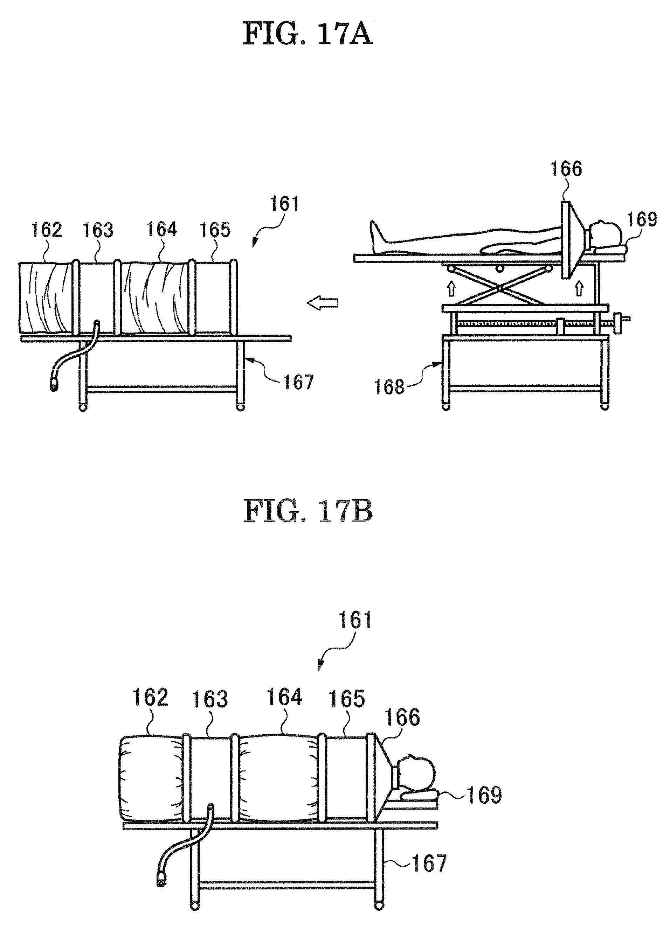

An envelope body 161 illustrated in Fig. 17A, Fig. 17B is one for whole body except for a head. The envelope body 161 has a bag shape, and is formed in a so-called capsule shape. A supply space to which carbon dioxide is supplied is formed in the envelope body 161. The envelope body 161 is formed of a plurality of (five) envelope members 162, 163, 164, 165, 166. A material of the envelope members 162, 164, 166 employs a chloroprene rubber and the like, for example. Further, a material of the envelope members 163, 165 employs a transparent acrylic as a casing, for example. The envelope member 166 has a similar structure to that of the envelope member 152 illustrated in Fig. 15. Here, the envelope body 161 is fixed to a mounting table 167, as illustrated in Fig. 17A and Fig. 17B.

Next, explanation will be made on a method in which the whole body is accommodated in the envelope body 161, with reference to Fig. 17A. First, a user exposes his/her head to the outside from the envelope member 166. Next, the user lies down on a stretcher 169 mounted on a carrier 168, as illustrated in Fig. 17A. Next, the carrier 168 is transferred to an opening of the envelope member 165, and is moved into the envelope members 162, 163, 164, 165 in a state where the user is placed on the stretcher 169. Next, the user connects the envelope member 165 and the envelope member 166. Fig. 17B is a diagram illustrating a state where the envelope member 165 and the envelope member 166 are connected and the whole body except for the head is enveloped by the envelope body 161.
Note that as another form of the envelope body, it is also possible to use the envelope body in the first to the third embodiments.

As described above, according to the present embodiment, there is used a large-sized supply unit (large-sized cylinder) capable of accommodating the carbon dioxide to be supplied into the body in a very highly compressed state. Therefore, it is possible to discharge a large amount of carbon dioxide, resulting in that the carbon dioxide can be supplied to a wide range of the surface of the body such as the whole body, for example.
Further, in the present embodiment, it is designed such that the permeable film member through which carbon dioxide permeates is wrapped around the body over the surface of the body on which the absorbent material is put or the body surface to which the absorbent material is applied. Therefore, the pressurized carbon dioxide presses the permeable film member wrapped around the body. The pressing force realizes a close contact between the absorbent material and the body surface, resulting in that the efficiency for making the carbon dioxide to be absorbed into the body from the body surface can be further improved.

### (Fifth Embodiment)

A fifth embodiment is an embodiment of a case where an absorbent body accommodating a gel-type absorbent material is integrally attached to an envelope body.

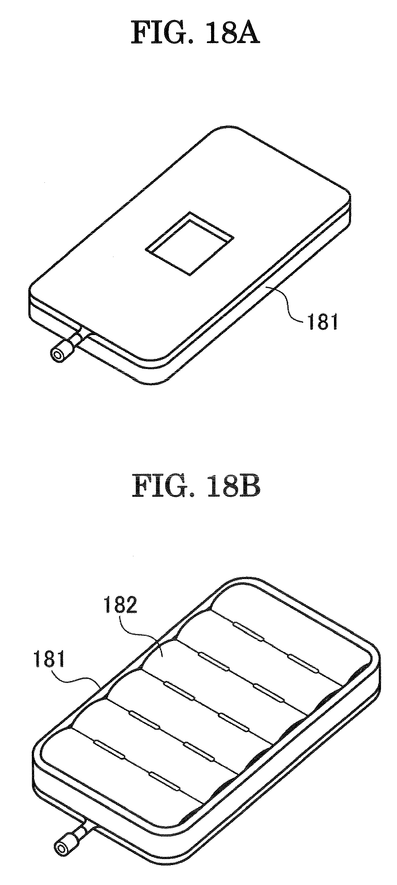

Fig. 18A, Fig. 18B illustrate a structure of the envelope body to which the absorbent body according to the fifth embodiment is integrally attached. Fig. 18A is a diagram in which an envelope body 181 is seen from a front side. Fig. 18B is a diagram in which the envelope body 181 is seen from a back side. As illustrated in Fig. 18B, the envelope body 181 is structured such that an absorbent body 182 can be integrally attached thereto.

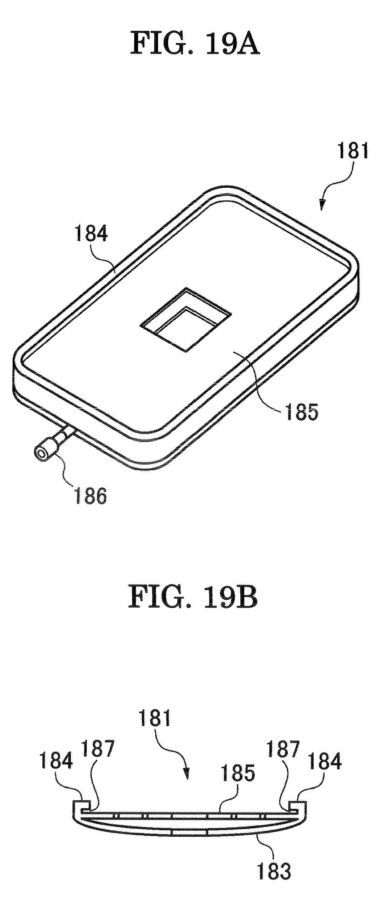

The envelope body 181 will be described with reference to Fig. 19A, Fig. 19B. The envelope body 181 includes an envelope member 183, a peripheral edge portion 184, a partition member 185, and a suction port 186. The envelope body 181 of the present embodiment is a member that is applied to a body surface of a lumbar, a back or the like, for example, and seals the inside of the envelope body 181, thereby enveloping the body surface. The envelope body 181 illustrated in Fig. 19A is illustrated so that a surface which is brought into contact with the body surface can be seen. Fig. 19B illustrates a cross section of the envelope body 181.

The envelope member 183 is formed in a sheet shape. A material of the envelope member 183 of the present embodiment employs a chloroprene rubber and the like, for example. Note that on a center portion of the envelope member 183, there is provided a transparent member through which a user can visually recognize the inside of the envelope body 181.
The peripheral edge portion 184 is standingly provided along an outer periphery of the envelope member 183. The peripheral edge portion 184 uses a material with sealing property, and uses, for example, the chloroprene rubber. Here, by applying the envelope body 181 to at least a part of the body surface, a supply space to which carbon dioxide is supplied is formed in a portion surrounded by the envelope member 183, the peripheral edge portion 184, and the body surface.

The partition member 185 is provided between the envelope member 183 and the peripheral edge portion 184, so as to partition the supply space into the body surface side and the envelope body side. As the partition member 185, there is used a material having permeability to gas, and elasticity such as, for example, a wrap film on which holes are formed, a fine mesh nonwoven fabric and the like. Note that on a center portion of the partition member 185, there is provided a transparent member through which the user can visually recognize the inside of the envelope body 181. Further, there is formed, between the partition member 185 and the peripheral edge portion 184, a gap portion 187 in which an end portion of the absorbent body 182 can be fitted.
The suction port 186 has a hole communicated with a space between the envelope member 183 and the partition member 185, and it can supply carbon dioxide, through the hole, to the space between the envelope member 183 and the partition member 185.

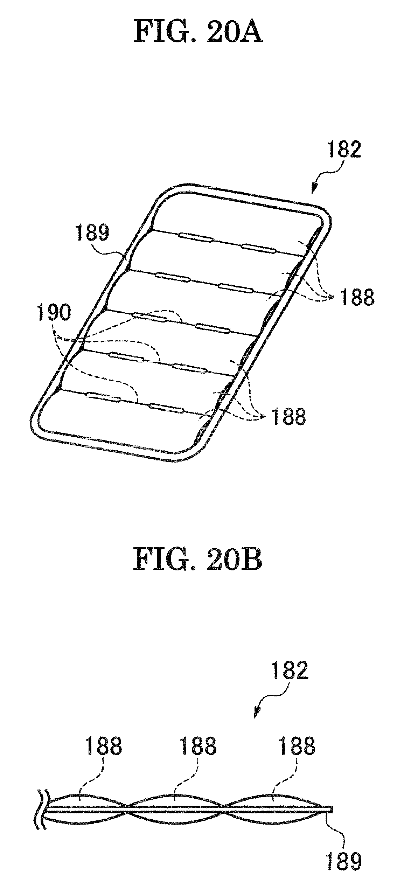

The absorbent body 182 will be described with reference to Fig. 20A, Fig. 20B. The absorbent body 182 is formed to have a size capable of being fitted in a space between the partition member 185 and the peripheral edge portion 184. The absorbent body 182 illustrated in Fig. 20A is illustrated so that a surface which is brought into contact with the body surface can be seen. Fig. 20B illustrates a side view of the absorbent body 182. As the absorbent body 182, there is used a material having permeability to gas, and elasticity such as, for example, a nonwoven fabric. Further, the absorbent body 182 is structured such that an inside thereof is divided into a plurality of (six) spaces 188. In the spaces, gel-type absorbent materials are packed. Therefore, as illustrated in Fig. 20B, the absorbent body 182 is formed in a concave and convex shape in which each space in which the absorbent material is packed bulges. A transfer path 190 is formed between the space and the space of the absorbent body 182 so that the absorbent materials packed in the respective spaces can move through the respective spaces. As described above, the absorbent body 182 has the concave and convex shape, and the gel-type absorbent materials packed in the spaces can move through the respective spaces, so that even when it is applied to an uneven surface of the body, it can be deformed to match the unevenness.

Further, on a surface on the body surface side of the absorbent body 182, coarse mesh holes or a large number of holes are formed so that the gel-type absorbent material packed in the space is easily discharged to the body surface side from the absorbent body 182. Meanwhile, to a surface on the envelope member side of the absorbent body 182, fine mesh holes or a small number of holes are formed so that the gel-type absorbent material is not leaked to the envelope member side while keeping the permeability of gas.
Further, an end portion 189 on an outer periphery of the absorbent body 182 is formed to have a thickness capable of being fitted in the gap portion 187 between the partition member 185 and the peripheral edge portion 184 of the envelope body 181. Therefore, it is structured such that the absorbent body 182 is detachable with respect to the envelope body 181 by attaching/detaching the absorbent body 182 to/from the gap portion 187 between the partition member 185 and the peripheral edge portion 184 of the envelope body 181.

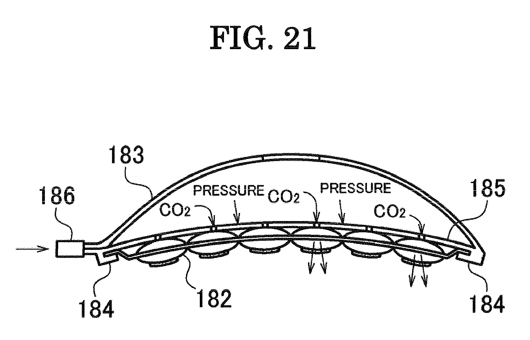

Next, explanation will be made on a method of supplying carbon dioxide to the envelope body 181 attached to the surface of the body. Note that as the supply unit that supplies carbon dioxide to the envelope body 181, it is assumed to use the handy-type cylinder or the large-sized cylinder explained in the second and the fourth embodiments. First, the user applies the envelope body 181 to which the absorbent body 182 is attached to the surface of the body, and fixes it to the body so as to seal the inside of the envelope body 181. At this time, as described above, the absorbent body 182 is deformed to a shape that matches unevenness of the surface of the body, which realizes a close contact between the absorbent body 182 and the body surface. Next, the user uses a suction tube to connect the supply unit to the suction port 186 of the envelope body 181. Further, the user opens the knob portion of the supply unit. Then, the carbon dioxide is discharged from the supply unit into the space between the partition member 185 and the envelope member 183 in the envelope body 181. The supplied carbon dioxide is uniformly filled in the space between the partition member 185 and the envelope member 183. At this time, the space between the envelope member 183 and the partition member 185 of the envelope body 181 expands as illustrated in Fig. 21. When the user keeps supplying the carbon dioxide, the carbon dioxide filled in the space between the envelope member 183 and the partition member 185 is compressed by the carbon dioxide which is continuously supplied. A large amount of the pressurized carbon dioxide permeates through the partition member 185, further permeates through the surface on the envelope member side of the absorbent body 182, and efficiently dissolves in the absorbent material packed in the space of the absorbent body 182. The absorbent material in which a large amount of carbon dioxide is dissolved is discharged from the body surface side of the absorbent body 182, and a larger amount of carbon dioxide can be absorbed into the body from the body surface closely contacted with the absorbent body 182. Further, the absorbent body 182 is pressed against the body surface side by the carbon dioxide pressurized in the space between the partition member 185 and the envelope member 183. Therefore, the pressing force can make the absorbent body 182 deform to match the unevenness of the body surface, resulting in that the efficiency for making the carbon dioxide to be absorbed from the surface of the body can be improved.

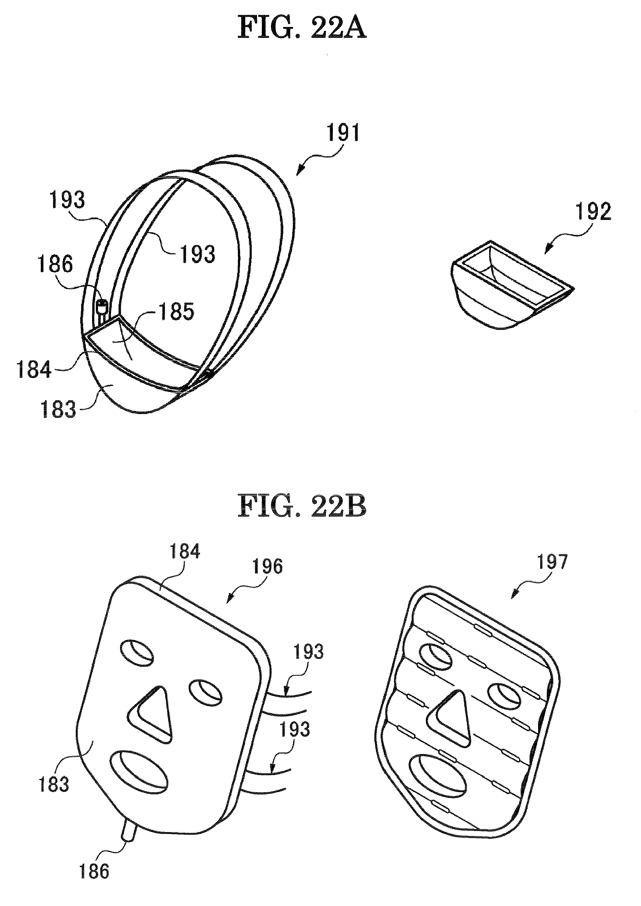

Next, another form of the envelope body used in the present embodiment will be described with reference to Fig. 22A to Fig. 22C. An envelope body 191 illustrated in Fig. 22A is one for chin. An envelope member 183 of the envelope body 191 is formed in a concave shape to correspond to a shape of the chin. Further, on both end portions of the envelope member 183, for example, belt-shaped fixing portions 193 made of rubber with elasticity are provided. A user can attach the envelope body 191 to his/her chin by making the belt-shaped fixing portions 193 of the envelope member 183 to be positioned at the back of his/her head. An absorbent body 192 has a size capable of being fitted in a space between the envelope member 183 and a peripheral edge portion 184, and is formed in a concave shape to correspond to the shape of the chin.

An envelope body 196 illustrated in Fig. 22B is one for face. An envelope member 183 of the envelope body 196 is formed to correspond to a shape of the face, and includes opening portions at portions corresponding to eyes, a nose and a mouth. Further, on both end portions of the envelope member 183, belt-shaped fixing portions 193 made of rubber are provided. An absorbent body 197 has a size capable of being fitted in a space between the envelope member 183 and a not-illustrated peripheral edge portion, and includes opening portions at portions corresponding to the eyes, the nose and the mouth, similar to the envelope body 196.

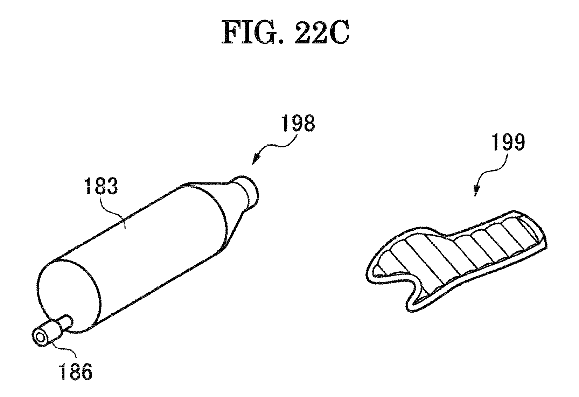

An envelope body 198 illustrated in Fig. 22C is one for a hand including a part of lower arm. An envelope member 183 of the envelope body 198 has a bag shape, and has an insertion opening formed thereon from which the arm can be inserted. Note that a not-illustrated partition member is integrally provided in a bag shape on the inside of the envelope member 183. An absorbent body 199 has a size capable of being fitted in a space formed by the partition member, and is formed to have a bag shape so as to correspond to a shape of the hand including a part of the lower arm.

Note that in the present embodiment, a case where the absorbent body including the gel-type absorbent material is used is explained, but, the present invention is not limited to this case. It is also possible to use the sheet-shaped absorbent material in Fig. 8A as the absorbent material. By using the sheet-shaped absorbent material suitable for mass production, it is possible to manufacture the absorbent material at a low cost.

As above, according to the present embodiment, it is designed such that the absorbent body accommodating the gel-type absorbent material is integrally attached to the envelope body. Therefore, the user does not have to make the gel-type absorbent material adhere to the body surface or to apply the absorbent material to the body surface, which improves usability.
Further, in the present embodiment, the absorbent body is formed to have the concave and convex shape so that the gel-type absorbent materials packed in the spaces of the absorbent body can move through the respective spaces, so that even when it is applied to an uneven surface of the body, it can be deformed to match the unevenness. Therefore, the absorbent body 182 is deformed to a shape that matches the unevenness of the surface of the body, which realizes a close contact between the absorbent body 182 and the body surface, resulting in that the efficiency for making the carbon dioxide to be absorbed into the body surface from the gel-type absorbent material in which the carbon dioxide is dissolved, can be improved.

### (Sixth Embodiment)

A sixth embodiment is an embodiment of a case where an adhesive portion is provided to an envelope body, and the envelope body is put on a body surface.

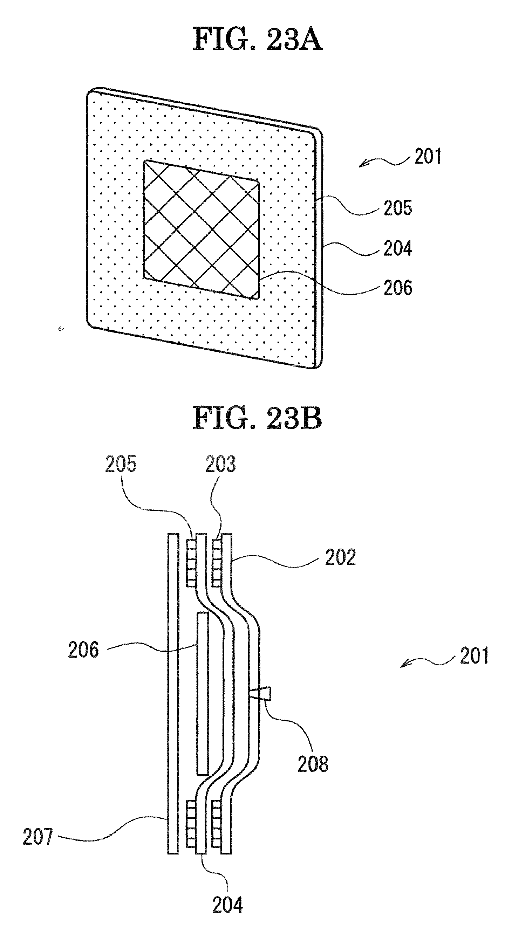

Fig. 23A, Fig. 23B illustrate a structure of an envelope body according to the sixth embodiment. Fig. 23A is a perspective view illustrating an external appearance of the envelope body. Further, Fig. 23B is a sectional view illustrating a structure of the envelope body.
As illustrated in Fig. 23B, an envelope body 201 includes a seal laminate 202, an adhesive sheet 204, a moisture retention mesh 206, antibacterial sheets 207, and a suction port 208. The envelope body 201 is formed in a sheet shape in which the seal laminate 202, the adhesive sheet 204, the moisture retention mesh 206, and the antibacterial sheets 207 are stacked.

First, the seal laminate 202 is an envelope member enveloping a part of the body surface, and is formed of a material such as rubber having impermeability to gas and capable of expanding and contracting. Further, to an outer edge side of a surface of the seal laminate 202 facing the adhesive sheet 204 except for a center portion, an adhesive portion for sealing 203 is provided. By the adhesive portion for sealing 203, the seal laminate 202 and the adhesive sheet 204 are adhered.

The adhesive sheet 204 is a partition member that partitions a later-described supply space and the body surface, and is formed to have substantially the same size as that of the seal laminate 202. The adhesive sheet 204 is formed so that gas can permeate therethrough, and is formed of a material having elasticity depending on a region of the body surface enveloped by the envelope body 201, such as a rubber having a plurality of holes, for example. Here, in a space surrounded by the seal laminate 202 and the adhesive sheet 204, there is formed the supply space to which carbon dioxide is supplied. Note that since the seal laminate 202 and the adhesive sheet 204 are adhered by the aforementioned adhesive portion for sealing 203, the carbon dioxide supplied to the supply space is prevented from being leaked to the outside between the seal laminate 202 and the adhesive sheet 204. Further, to a surface of the adhesive sheet 204 facing the antibacterial sheets 207 except for a center portion, an adhesive portion for body surface 205 is provided. The adhesive portion for body surface 205 has an adhesion for putting the envelope body 201 on the body surface. Note that to the adhesive portion for body surface 205, the antibacterial sheets 207 which are peeled off right before the adhesive portion for body surface 205 is put on the body surface, are adhered in a manner that they can be easily removed.

The moisture retention mesh 206 is formed to be smaller than the adhesive sheet 204, and is disposed on a center of the adhesive sheet 204. The moisture retention mesh 206 is formed of a gauze woven by cotton yarn or the like in which an absorbent material such as water, alcohols, oils and fats or the like, for example, being a medium in which carbon dioxide can be dissolved, is permeated, and serves as an absorbent part. Note that it is also possible that in the moisture retention mesh 206, nano-sized medicine, collagen or the like (referred to as nano-sized medicine or the like) having a DDS (Drug Delivery System) effect is made to permeate, together with the absorbent material in which carbon dioxide can be dissolved.

The antibacterial sheets 207 are formed to have substantially the same size as that of the adhesive sheet 204. The antibacterial sheets 207 can prevent vaporization of the absorbent material permeated in the moisture retention mesh 206, and prevent adhesion of dirt with respect to the moisture retention mesh 206.
The suction port 208 is provided on a center of the seal laminate 202. The suction port 208 has a hole communicated with the supply space, and can be connected to the blow-out port of the supply unit.

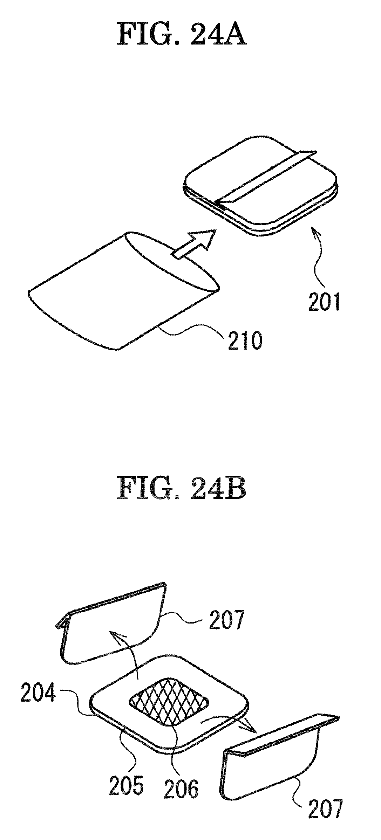

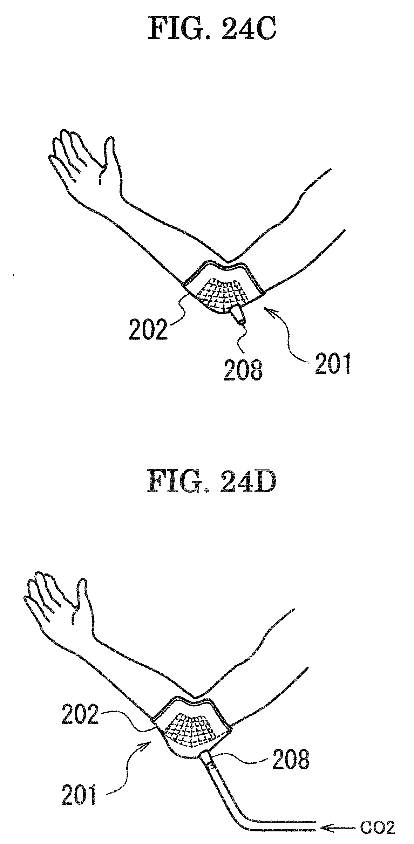

Next, explanation will be made on an attachment method of the envelope body 201 and a method of supplying carbon dioxide into the envelope body 201 using the supply unit, with reference to Fig. 24A to Fig. 24E. Here, explanation will be made by citing an elbow as a region of body on which the envelope body 201 is put, as an example. First, as illustrated in Fig. 24A, a user takes out the envelope body 201 from a portable case 210. Next, as illustrated in Fig. 24B, the user peels off the antibacterial sheets 207 adhered to the adhesive portion for body surface 205 of the adhesive sheet 204. Subsequently, as illustrated in Fig. 24C, the user puts on the envelope body 201 so that the moisture retention mesh 206 of the envelope body 201 faces a surface of the elbow. At this time, since the adhesive portion for body surface 205 of the envelope body 201 becomes in a state of being closely contacted with the elbow due to its adhesion, the moisture retention mesh 206 can be closely contacted with the body surface.

Next, as illustrated in Fig. 24D, the user connects the blow-out port of the supply unit of the aforementioned embodiment to the suction port 208 of the envelope body 201 put on the elbow. When the user discharges the carbon dioxide from the supply unit, the carbon dioxide is uniformly filled in the supply space surrounded by the seal laminate 202 and the adhesive sheet 204. When the user keeps supplying the carbon dioxide, the carbon dioxide filled in the supply space is compressed by the carbon dioxide which is continuously supplied. A large amount of the pressurized carbon dioxide permeates through the adhesive sheet 204, and efficiently dissolves in the absorbent material such as water permeated in the moisture retention mesh 206.
With the use of the absorbent material in which a large amount of carbon dioxide dissolves, it is possible to make a larger amount of carbon dioxide to be absorbed into the body from the body surface which is closely contacted with the moisture retention mesh 206. Further, the moisture retention mesh 206 is pressed against the body surface side by the carbon dioxide pressurized in the supply space. Therefore, the pressing force realizes a close contact between the moisture retention mesh 206 and the body surface, resulting in that the efficiency for making the carbon dioxide to be absorbed into the body from the body surface can be further improved. Note that when the nano-sized medicine or the like having the DDS effect is permeated in the moisture retention mesh 206, the nano-sized medicine or the like is absorbed into the body from the body surface together with the carbon dioxide, so that it can efficiently reach an affected part to be a target.

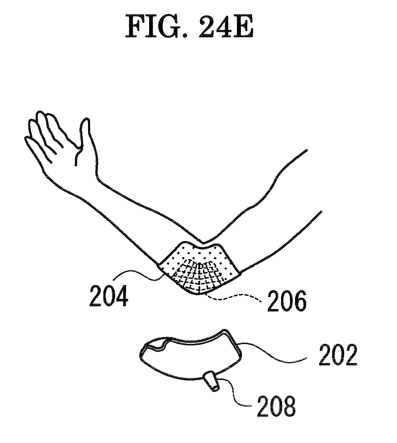

Next, when the supply of carbon dioxide is terminated, the user removes the envelope body 201 put on the elbow. Note that when there is a wound on the elbow on which the envelope body 201 is put, if the envelope body 201 is removed, the wound is exposed to the outside. Therefore, when there is a wound on the elbow on which the envelope body 201 is put, the user peels off only the seal laminate 202 from the adhesive sheet 204 of the envelope body 201, as illustrated in Fig. 24E. By peeling off the seal laminate 202 as above, the wound on the elbow is kept covered by the adhesive sheet 204, so that the wound is not exposed to the outside, and bacteria can be prevented from entering the wound from the outside.
Note that by forming the envelope body 201 in a size in accordance with the body surface on which the envelope body is put, the envelope body 201 can deal with various regions of body. Further, by using the material with elasticity for the seal laminate 202 and the adhesive sheet 204, even when the envelope body 201 is used for regions of body with complicated shapes such as the elbow, the shoulder, the joint of knee and the like, the chin, the forehead and the like, it is possible to put on the envelope body 201 along the body surface.

As above, according to the present embodiment, there is provided the adhesive portion for body surface to the envelope body. Therefore, the user can easily put the envelope body on the body surface, which improves usability.
Further, in the present embodiment, even if there is a wound on the region enveloped by the envelope body, by peeling off only the seal laminate stacked on the outside of the envelope body, the adhesive sheet covers the wound, resulting in that bacteria can be prevented from entering the wound from the outside.

### (Seventh Embodiment)

A seventh embodiment is an embodiment of a case where bubble carbon dioxide is supplied as mousse into an envelope body enveloping at least a part of body surface.

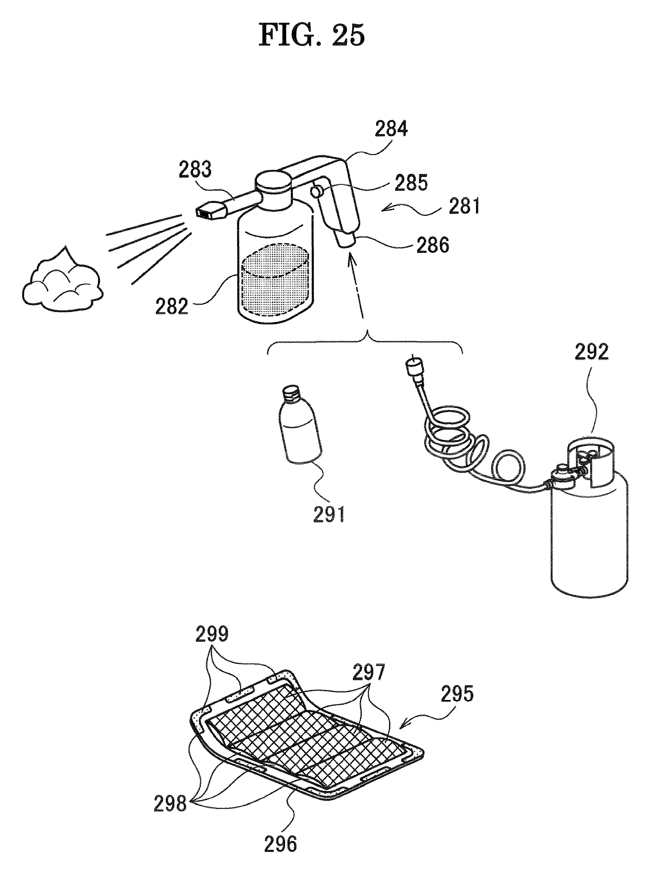

Fig. 25 illustrates a structure of a carbon dioxide supply device according to the seventh embodiment. As illustrated in Fig. 25, the carbon dioxide supply device includes a supply unit 281 and an envelope body 295.

The supply unit 281 is a so-called spray gun, and includes a container body 282, an injection port 283, a handle portion 284, an injection switch 285, and an insertion port 286. The container body 282 accommodates a foamable gel, as an absorbent material to be adhered to the body surface. Here, the foamable gel as the absorbent material is liquid (medium) containing components suitable for the generation of foam, and in which carbon dioxide can be dissolved.

As illustrated in Fig. 25, it is structured such that a cartridge 291 or a nozzle of cylinder 292 can be connected to the insertion port 286. Here, in the cartridge 291, carbon dioxide is compressed to be accommodated. Further, in the cylinder 292, carbon dioxide is highly compressed and accommodated in a liquefied state. To the cylinder 292, there are provided a pressure reducer, a scale portion, a cock portion and the like, and can discharge carbon dioxide in a gaseous state by reducing pressure to a desired pressure.
When a user attaches the cartridge 291 or the nozzle of cylinder 292 to the insertion port 286, and then pushes down the injection switch 285 provided to the handle portion 284, the supply unit 281 foams the foamable gel to generate a mousse containing bubble carbon dioxide, and injects the mousse from the injection port 283. In the injected mousse, the bubble is carbon dioxide, and the other is the absorbent material.

The envelope body 295 includes an envelope member 296, permeable members 297, filling portions 298, and adhesive portions 299. The envelope member 295 has a shape capable of enveloping the arm, and is formed of, for example, polyvinyl chloride, nylon, a synthetic resin, a cloth or the like having flexibility. The envelope material 296 preferably uses a material through which the mousse does not permeate to the outside.

On an inner peripheral surface of the envelope member 296, meshed permeable members 297 are attached over substantially the whole surface of the envelope member 296. A plurality of pocket-shaped filling portions 298 in which the mousse can be filled are formed as mousse accommodating portions between the permeable members 297 and the envelope member 296. The mousse filled in the filling portion 298 can permeate through the meshed permeable member 297. Further, on an outer edge of the inner peripheral surface of the envelope member 296, the adhesive portions 299 are provided. When the adhesive portions 299 become in a state of being closely contacted with the arm due to their adhesion, the inner peripheral surface of the envelope member 296 and the body surface can be closely contacted with each other.

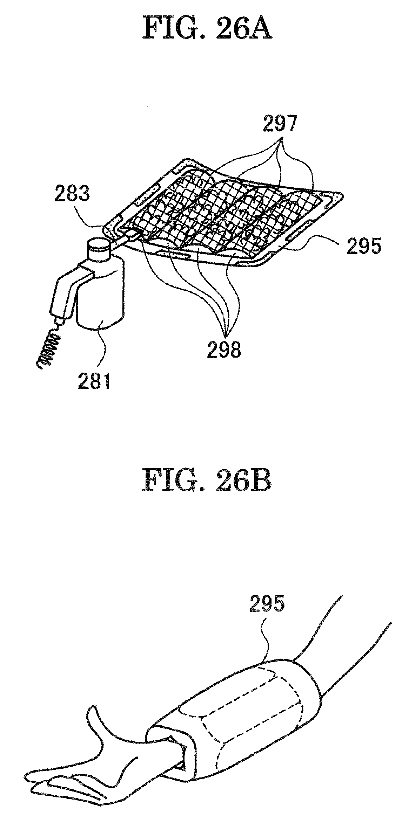

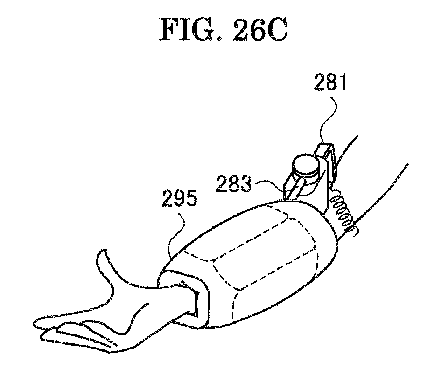

Next, an attachment method of the envelope body 295 and a method of supplying carbon dioxide into the envelope body 295 using the supply unit 281 will be described with reference to Fig. 26A to Fig. 26C. First, as illustrated in Fig. 26A, a user pushes down the injection switch 285 in a state where the injection port 283 of the supply unit 281 faces the pocket-shaped filling portion 298, thereby filling the filling portion 298 with the mousse. After uniformly filling all of the filling portions 298 with the mousse, the user wraps the envelope body 295 around the body surface so that the inner peripheral surface of the envelope body 295 faces the body surface, as illustrated in Fig. 26B. At this time, due to the adhesion of the adhesive portions 299 of the envelope body 295, the envelope member 296 and the body surface are closely contacted with each other. Next, as illustrated in Fig. 26C, the user inserts the injection port 283 of the supply unit 281 between the envelope body 295 and the body surface to further fill the filling portion 298 with the mousse, thereby pressurizing the filled mousse.

In the envelope body 295, the mousse filled in the filling portion 298 permeates through the permeable member 297 to be adhered to the body surface. Meanwhile, in the mousse, the bubble carbon dioxide gradually dissolves in the absorbent material. Therefore, the absorbent material in which the carbon dioxide dissolves is absorbed into the body from the body surface. Note that as illustrated in Fig. 26C, by further filling the inside of the envelope body 295 with the mousse, it is possible to pressurize the mousse filled in the envelope body 295, resulting in that a large amount of bubble carbon dioxide can be efficiently dissolved in the absorbent material. With the use of the absorbent material in which a large amount of carbon dioxide dissolves, it is possible to make a larger amount of carbon dioxide to be absorbed into the body from the body surface. Further, by being pressurized, the absorbent material is pressed against the body surface. Therefore, it is possible to improve the efficiency for making the carbon dioxide to be absorbed into the body from the body surface. Further, it is also possible to mix the nano-sized medicine or the like having the DDS effect described above to the mousse to be generated.

As time passes, the mousse positioned close to the body surface is gradually liquefied by being warmed at a skin temperature. Accordingly, the absorbent material liquefied at the position close to the body surface runs downward due to gravity, and an amount of mousse in the envelope body 295 is reduced, but, in accordance with that, the mousse positioned on the outside moves to the body surface side. In the mousse positioned on the outside, the bubble carbon dioxide is uniformly contained, so that even when the amount of mousse is reduced, it is possible to make fresh carbon dioxide to be dissolved in the absorbent material. Note that by directly applying the mousse to the skin, not by filling the envelope body with the mousse, it is possible to obtain the similar effect.
As above, according to the present embodiment, by making the bubble carbon dioxide to be contained in the mousse and adhered to the body surface, it is possible to make the carbon dioxide to be absorbed into the body through the absorbent material.

### (Eighth Embodiment)

An eighth embodiment is an embodiment of a case where a concentration adjusting unit adjusting a concentration of carbon dioxide is added to a supply unit supplying carbon dioxide into an envelope body enveloping at least a part of body surface. The concentration adjusting unit of the present embodiment requires no power supply, so that even in a place where it is not possible to supply power when carrying a carbon dioxide supply device, the concentration adjusting unit operates. Note that the reason why the concentration of carbon dioxide is adjusted is because a speed and an amount of absorbing carbon dioxide become different depending on a region of body or individual difference, and by setting the concentration of carbon dioxide to one in accordance with the region of body or each person, it is possible to bring out the Bohr effect in accordance with each person.

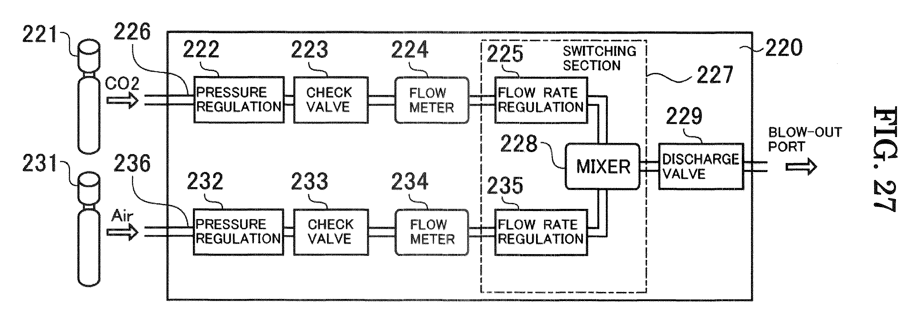

Fig. 27 illustrates a structure of the concentration adjusting unit according to the eighth embodiment. Fig. 27 is a diagram illustrating an example of the structure of the concentration adjusting unit.
As illustrated in Fig. 27, to a concentration adjusting unit 220, carbon dioxide is supplied from a supply unit 221, and air is supplied from a high pressure cylinder 231. The concentration adjusting unit 220 includes a path 226 through which the carbon dioxide is supplied and a path 236 through which the air is supplied, pressure regulating parts 222, 232, check valves 223, 233, flow meters 224, 234, flow rate regulating parts 225, 235, a mixer 228, and a discharge valve 229. Note that the flow rate regulating parts 225, 235 and the mixer 228 form a switching section 227.

In the pressure regulating parts 222, 232, pressures of the carbon dioxide and the air inflowed from the supply unit 221 and the high pressure cylinder 231 are regulated. At this time, a user performs regulation so that the pressures of the carbon dioxide and the air become equal, in each of the pressure regulating parts 222, 232. Next, in the check valves 223, 233, a back flow of the inflowed carbon dioxide and air is prevented. Next, in the flow meters 224, 234, flow rates of the inflowed carbon dioxide and air can be checked by the user. Next, the flow rate regulating parts 225, 235 adjust the concentration of carbon dioxide by changing the flow rates of the carbon dioxide and the air. Next, in the mixer 228, the carbon dioxide and the air whose flow rates are changed in the flow rate regulating parts 225, 235 are mixed, and concentration-adjusted carbon dioxide is generated. At last, in the discharge valve 229, the concentration-adjusted carbon dioxide is discharged when the valve is opened. The concentration-adjusted carbon dioxide is supplied into an envelope body.

Note that the user can adjust the concentration of carbon dioxide not only by changing the flow rates of the carbon dioxide and the air in each of the flow rate regulating parts 225, 235, but also by using the switching section 227 formed of the flow rate regulating parts 225, 235, and the mixer 228. Concretely, when the user performs rotation operation and the like of an operation knob coupled to the switching section 227, it is possible to adjust the concentration of carbon dioxide, in a step-by-step manner, to 25, 50, 75 percent and the like, or to adjust the concentration of carbon dioxide in a stepless manner. Here, when it is structured to perform adjustment in a stepless manner, it is structured such that the concentration of carbon dioxide can be adjusted from approximately 1000 ppm to approximately 95 percent. Note that orders of the pressure regulating parts 222, 232 and the check valves 223, 233, orders of the check valves 223, 233 and the flow meters 224, 234 and the like may be exchanged to structure the concentration adjusting unit 220.

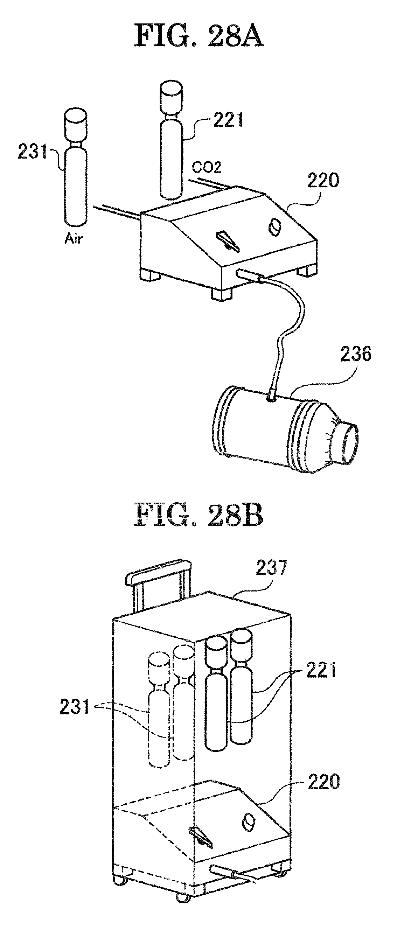

Next, an external appearance of the concentration adjusting unit of the present embodiment will be described with reference to Fig. 28A and Fig. 28B. Fig. 28A is an exterior view illustrating an example of a desk-top concentration adjusting unit. As illustrated in Fig. 28A, the concentration adjusting unit 220 is formed in a desk-top size. Further, the supply unit 221 and the concentration adjusting unit 220, and the high pressure cylinder 231 and the concentration adjusting unit 220 are connected with tubes. Further, the concentration adjusting unit 220 and an envelope body 236 are connected via a tube so that concentration-adjusted carbon dioxide discharged from the concentration adjusting unit 220 is supplied into the envelope body 236.
Fig. 28B is an exterior view illustrating an example of a carrying-type concentration adjusting unit. As illustrated in Fig. 28B, the concentration adjusting unit 220 is accommodated in a case 237 provided with casters. Further, in the case 237, a plurality of supply units 221 and high pressure cylinders 231 are also accommodated. As above, since the concentration adjusting unit 220 is accommodated in the case 237, it can be easily carried.

As above, according to the present embodiment, since the concentration adjusting unit adjusting the concentration of carbon dioxide supplied by the supply unit is added, by setting the concentration of carbon dioxide to one in accordance with the region of body or each person, it is possible to bring out the Bohr effect in accordance with each person.

### (Ninth Embodiment)

A ninth embodiment is an embodiment of a case where a concentration adjusting unit automatically adjusting a concentration of carbon dioxide is added to a supply unit supplying carbon dioxide into an envelope body enveloping at least a part of body surface. In the concentration adjusting unit of the ninth embodiment, a control circuit automatically performs the concentration adjustment.

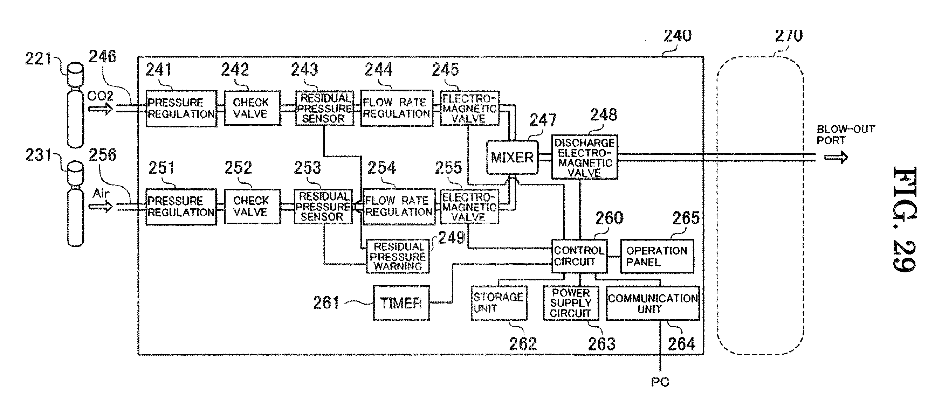

Fig. 29 illustrates a structure of the concentration adjusting unit according to the ninth embodiment. Fig. 29 is a diagram illustrating an example of a structure of the concentration adjusting unit. Note that out of components of a concentration adjusting unit 240 illustrated in Fig. 29, the same components as those of the concentration adjusting unit 220 according to the eighth embodiment will be denoted by the same names and detailed explanation thereof will be omitted.

As illustrated in Fig. 29, to the concentration adjusting unit 240, carbon dioxide is supplied from the supply unit 221, and air is supplied from the high pressure cylinder 231. The concentration adjusting unit 240 includes a path 246 through which the carbon dioxide is supplied and a path 256 through which the air is supplied, pressure regulating parts 241, 251, check valves 242, 252, residual pressure sensors 243, 253, flow rate regulating parts 244, 254, electromagnetic valves 245, 255, a mixer 247, and a discharge electromagnetic valve 248. Further, the concentration adjusting unit 240 includes a residual pressure warning unit 249, a control circuit 260, a timer 261, a storage unit 262, a power supply circuit 263, a communication unit 264, and an operation panel 265.

In the pressure regulating parts 241, 251, a user performs regulation so that pressures of the carbon dioxide and the air inflowed from the supply unit 221 and the high pressure cylinder 231 become equal. Next, in the check valves 242, 252, a back flow of the inflowed carbon dioxide and air is prevented. Next, in the residual pressure sensors 243, 253, the pressures of the inflowed carbon dioxide and air are measured. Pressure values measured by the residual pressure sensors 243, 253 are transmitted to the residual pressure warning unit 249. When the transmitted pressure value becomes equal to or less than a predetermined pressure value, the residual pressure warning unit 249 gives a warning that there is no remaining amount of the carbon dioxide or the air in the supply unit 221 or the high pressure cylinder 231. Next, the flow rate regulating parts 244, 254 adjust the concentration of carbon dioxide by changing the flow rates of the carbon dioxide and the air. Further, the electromagnetic valves 245, 255 also adjust the concentration of carbon dioxide by changing the flow rates of the carbon dioxide and the air, in the similar manner. Note that the concentration adjustment of the present embodiment is solely conducted by the electromagnetic valves 245, 255, and the flow rate regulating parts 244, 254 perform supplementary adjustment. The concentration adjusting unit 240 of the present embodiment is structured such that it can adjust the concentration of carbon dioxide from approximately 1000 ppm to approximately 95 percent.

Next, in the mixer 247, the carbon dioxide and the air whose flow rates are changed in the electromagnetic valves 245, 255 are mixed, and concentration-adjusted carbon dioxide is generated. At last, in the discharge electromagnetic valve 248, the concentration-adjusted carbon dioxide is discharged when the valve is opened.
Here, the electromagnetic valves 245, 255 and the discharge electromagnetic valve 248 are connected to the control circuit 260. Specifically, when the control circuit 260 controls the electromagnetic valves 245, 255, the flow rates of the carbon dioxide and the air are changed, resulting in that the concentration of carbon dioxide is adjusted. Further, when the control circuit 260 controls opening/closing of the discharge electromagnetic valve 248, it is possible to discharge the concentration-adjusted carbon dioxide or to stop the discharge.

Further, to the control circuit 260, the timer 261 and the operation panel 265 are connected. Accordingly, when the user sets, by using the operation panel 265, a period of time during which the carbon dioxide is continuously discharged, and the concentration of carbon dioxide, the control circuit 260 controls, based on the set period of time and concentration, the electromagnetic valves 245, 255 to perform control to achieve the set concentration, and can stop, by using the timer 261, the discharge of carbon dioxide by closing the discharge electromagnetic valve 248 when the set period of time elapses.

Further, the storage unit 262 is connected to the control circuit 260. It is possible to make the storage unit 262 store a plurality of programs to be executed by the control circuit 260. Here, the program is for making the control circuit 260 execute a menu such that, for example, carbon dioxide whose concentration is reduced is discharged for the first several minutes, carbon dioxide is then discharged for several minutes while gradually increasing the concentration of carbon dioxide, and the discharge of carbon dioxide is stopped after a given period of time elapses. As described above, the speed and the amount of absorbing carbon dioxide become different depending on the region of body or individual difference, so that by making the programs in accordance with the region of body in which carbon dioxide is tried to be absorbed, and the user, to be stored in the storage unit 262, and when the user operates the operation panel 265 to select the corresponding program from the plurality of programs stored in the storage unit 262, and the control circuit 260 executes the program, it is possible to bring out the Bohr effect in accordance with the region of body or the user.

Further, the concentration adjusting unit 240 is provided with the power supply circuit 263. Therefore, by inserting a plug connected to the power supply circuit 263 into a socket and the like, it is possible to supply power to drive the concentration adjusting unit 240. Further, the concentration adjusting unit 240 is provided with the communication unit 264. It is structured such that the communication unit 264 can be connected to a personal computer (PC), for example. Therefore, by transmitting data as a result of adjusting the concentration of carbon dioxide to the personal computer from the concentration adjusting unit 240, the personal computer can store the adjustment data of concentration in accordance with time axis. When the adjustment data of concentration of carbon dioxide is stored as above, it is possible to refer to the stored adjustment data when creating the program in accordance with the region of body or the user.
Note that in addition to the case where the aforementioned program in accordance with the region of body or the user is stored in the storage unit 262, it is also possible to structure such that the program is transmitted to the concentration adjusting unit 240 from the personal computer connected to the communication unit 264. Further, it is also possible to structure such that the concentration adjusting unit 240 can be controlled from the personal computer connected to the communication unit 264.

Further, in the drawing illustrated in Fig. 29, a sub-tank 270 for temporarily storing the concentration-adjusted carbon dioxide discharged from the concentration adjusting unit 240, is illustrated by a dotted line. The sub-tank 270 is used in a case where an envelope body is large, and a capacity of supply space to which carbon dioxide is supplied is large. Specifically, when carbon dioxide is supplied to the supply space with large capacity, it takes a long time to uniformly fill the inside of the supply space with carbon dioxide. Accordingly, by previously making the sub-tank 270 store the carbon dioxide discharged from the concentration adjusting unit 240, it is possible to supply the carbon dioxide from the sub-tank 270 to the supply space of the envelope body at a time, which enables to reduce the period of time for supplying the carbon dioxide into the supply space. Further, by structuring such that the carbon dioxide supplied into the supply space from the sub-tank 270 can be returned to the sub-tank 270, it is possible to supply the carbon dioxide to the supply space from the sub-tank 270 again, resulting in that the carbon dioxide can be reused. At this time, it is only required that a concentration measuring unit of carbon dioxide is provided in the sub-tank 270 to measure whether a concentration satisfies a predetermined concentration, and when the concentration does not satisfy the predetermined concentration, the carbon dioxide is filled in the sub-tank 270 from the concentration adjusting unit 240. This sub-tank 270 is not limited to the present embodiment, and it may also be provided at a position after the carbon dioxide is discharged from the concentration adjusting unit 220 of the eighth embodiment or to the blow-out port of the large-sized cylinder of the fourth embodiment.

Note that in the aforementioned concentration adjusting unit 240, it is also possible to structure such that the control circuit 260 can also perform control of pressure regulation by using electromagnetic valves in the pressure regulating parts 241, 251. For example, by creating a program such that the pressure regulation of the carbon dioxide and the air is changed, the pressure of carbon dioxide applied to the body surface can be changed, so that a massage effect with respect to the body surface can be expected. Further, it is also possible to structure such that the control circuit 260 also performs control of temperatures of the carbon dioxide and the air by conducting the pressure regulation. For example, by creating a program such that the pressure regulation of the carbon dioxide and the air is changed, the temperature of the concentration-adjusted carbon dioxide discharged from the concentration adjusting unit 240 can be changed, so that an effect to cool down the body surface and the like can be expected.

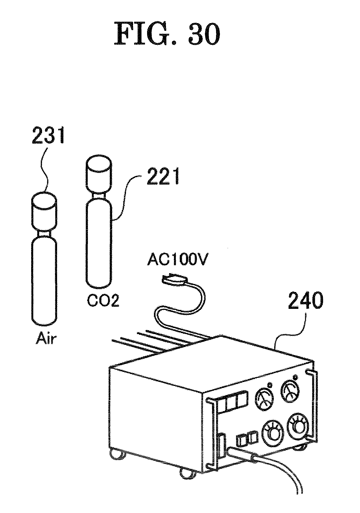

Next, an external appearance of the concentration adjusting unit of the present embodiment will be described with reference to Fig. 30. Fig. 30 is an exterior view illustrating an example of the concentration adjusting unit. As illustrated in Fig. 30, the supply unit 221 and the concentration adjusting unit 240, and the high pressure cylinder 231 and the concentration adjusting unit 240 are connected via tubes. Further, in order to make concentration-adjusted carbon dioxide discharged from the concentration adjusting unit 240 to be supplied into a not-illustrated envelope body, the concentration adjusting unit 220 and the envelope body are connected via a tube.

As above, according to the present embodiment, the concentration adjusting unit automatically adjusting the concentration of carbon dioxide supplied by the supply unit is added, so that by setting the concentration of carbon dioxide to one in accordance with the region of body or the user, it is possible to bring out the Bohr effect in accordance with each person. Further, by creating a program in accordance with the region of body or the user, and making the concentration adjusting unit operate based on the program, it is possible to further bring out the Bohr effect in accordance with the region of body or the user.

Note that as the supply unit 221 used in the eighth embodiment and the ninth embodiment, it is possible to use any one of the spray can in the first embodiment, the handy cylinder in the second embodiment, the large-sized cylinder in the fourth embodiment and the like. Further, the unit of supplying air is not limited to the high pressure cylinder 231, and may also be a compressor (compression machine), a handy cylinder and the like. Further, in the eighth embodiment and the ninth embodiment, the case where the gas to be mixed with carbon dioxide is air is explained, but, the present invention is not limited to this case, and it is also possible to use, for example, nitrogen gas, helium gas, radon gas and the like. Further, the concentration adjusting unit according to the eighth embodiment and the ninth embodiment can be added to the carbon dioxide supply device according to the first embodiment to the seventh embodiment described above.

### (Tenth Embodiment)

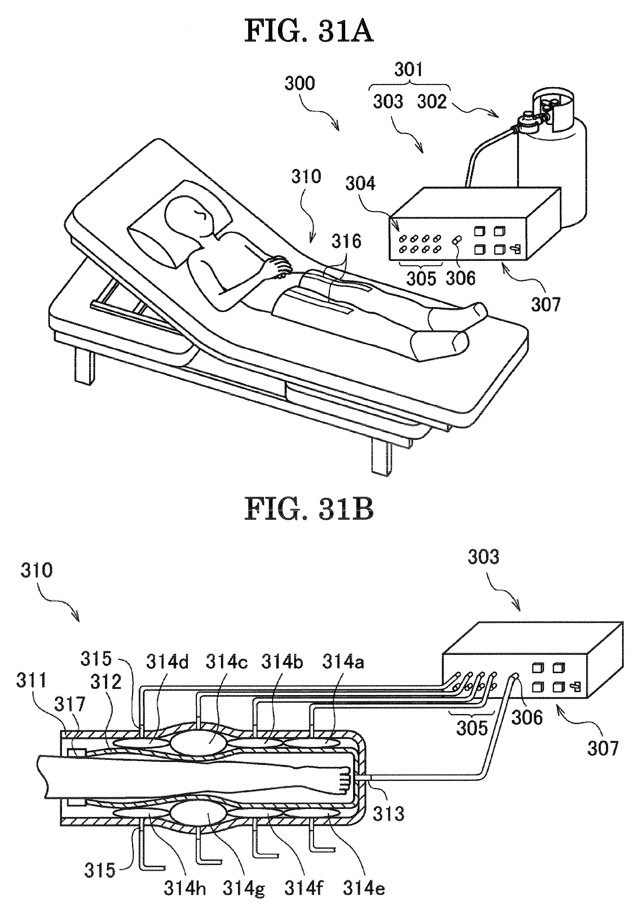

A tenth embodiment is another form of the carbon dioxide supply device according to the third embodiment. Hereinafter, a structure of a carbon dioxide supply device 300 according to the tenth embodiment will be described with reference to Fig. 31A, Fig. 31B. Fig. 31A is a diagram illustrating a structure of the entire carbon dioxide supply device 300. Fig. 31B is a diagram illustrating a cross section of a part of an envelope body 310.
The carbon dioxide supply device 300 includes an envelope body 310 and a supply and pressurizing unit 301 having both functions of supply unit and pressurizing unit.

The supply and pressurizing unit 301 includes a cylinder 302 in which carbon dioxide is highly compressed and accommodated in a liquefied state, and a supply and pressurization control unit 303 supplying carbon dioxide into the envelope body 310. The supply and pressurization control unit 303 includes a plurality of blow-out ports 304 and a plurality of switches 307. The supply and pressurization control unit 303 is connected to the cylinder 302, and can control a timing at which the carbon dioxide accommodated in the cylinder 302 is supplied into the envelope body 310 from the plurality of blow-out ports 304. The blow-out ports 304 include a carbon dioxide blow-out port 306 supplying carbon dioxide into a later-described partition member 312 of the envelope body 310, and gas blow-out ports 305 supplying gas into later-described gas bags 314a to 314h of the envelope body 310. Note that the carbon dioxide accommodated in the cylinder 302 is discharged as gas from the gas blow-out port 305 of the present embodiment, but, the gas is not limited to carbon dioxide, and it is also possible to structure such that air is discharged. Further, the supply and pressurization control unit 303 can change the timing at which the gas is supplied and the like, in accordance with the pushing-down of the plurality of switches 307.

Next, the envelope body 310 is one for legs including femora, crura and feet. The envelope body 310 is structured by including an envelope member 311, a partition member 312, a plurality of gas bags 314a to 314h and the like. The envelope member 311 employs a material that is resistant to expansion of the plurality of gas bags 314a to 314h. The partition member 312 partitions a supply space into the body surface side and the envelope body side inside the envelope member 311. The partition member 312 illustrated in Fig. 31B is formed to have a bag shape so as to cover the entire legs inside the envelope member 311. As the partition member 312, there is used a material having impermeability to gas, and elasticity such as, for example, a chloroprene rubber and the like. Further, to the partition member 312, there is provided a suction port 313 through which carbon dioxide is supplied into the partition member 312 via a tube from the carbon dioxide blow-out port 306 of the supply and pressurization control unit 303.

The plurality of gas bags 314a to 314h are arranged to be adjacent to one another along a longitudinal direction of the envelope body 310 on an outside of the partition member 312, namely, in a space between the envelope member 311 and the partition member 312. To the plurality of gas bags 314a to 314h, there are provided a plurality of suction ports 315 for supplying gas into the respective gas bags 314a to 314h via tubes from the gas blow-out ports 305 of the supply and pressurization control unit 303.

Next, explanation will be made on a method in which the user attaches the envelope body 310. First, the user applies the gel-type absorbent material to a surface of the entire legs including the femora, the crura and the feet. Note that it is also possible to put a substance such as a cloth in which a medium such as water, alcohols, oils and fats or the like in which carbon dioxide can be dissolved is permeated, on the surface of the entire legs. Next, the user opens airtight fasteners 316 of the envelope body 310 (refer to Fig. 31A), inserts the entire legs into the envelope member 311 and the partition member 312, and closes the airtight fasteners 316. At this state, it is designed such that the gas filled in the space between the body surface and the partition member 312 is not leaked to the outside of the partition member 312.
Next, the user connects the carbon dioxide blow-out port 306 of the supply and pressurization control unit 303 to the suction port 313 of the envelope body 310 via the tube, and connects the gas blow-out ports 305 to the suction ports 315 of the respective gas bags 314a to 314h of the envelope body 310 via the tubes, thereby completing the attachment of the envelope body 310.

Next, when the user pushes down an operation start button of the supply and pressurization control unit 303, the supply and pressurization control unit 303 controls the timing at which the carbon dioxide is supplied into the partition member 312 and the timing at which the gas is supplied to the gas bags 314a to 314h based on the set program.
First, the supply and pressurization control unit 303 fills the inside of the partition member 312 with carbon dioxide. Then, the carbon dioxide filled in the partition member 312 is compressed by carbon dioxide which is further supplied later. Therefore, a large amount of the pressurized carbon dioxide efficiently dissolves in the absorbent material applied to the entire legs, resulting in that a larger amount of carbon dioxide can be absorbed from the surface of the entire legs.

Subsequently, the supply and pressurization control unit 303 supplies the gas into the gas bags 314a to 314h arranged in the space between the envelope member 311 and the partition member 103. First, the supply and pressurization control unit 303 expands the gas bags 314a, 314e positioned at the end of the foot, and then contracts the gas bags. Next, the supply and pressurization control unit 303 expands the gas bags 314b, 314f positioned at the crus, and then contracts the gas bags. As above, the supply and pressurization control unit 303 expands the gas bags 314a to 314d and 314b to 314h positioned at the end of the foot, the crus, the knee, and the femur in this order. When the gas bags 314a to 314h expand, the gas supplied to the gas bags 314a to 314h compresses the carbon dioxide in the partition member 312 via the partition member 312.

Therefore, a large amount of the pressurized carbon dioxide efficiently dissolves in the absorbent material applied to the entire legs. Through the absorbent material in which a large amount of carbon dioxide dissolves, it is possible to make a larger amount of carbon dioxide to be absorbed from the surface of the entire legs. At this time, the supply and pressurization control unit 303 expands the gas bags 314a to 314h to pressurize the legs, so that the user can achieve a massage effect. Further, the supply and pressurization control unit 303 expands the gas bags 314a to 314h in the order from the end of body toward the center of body, so that it is possible to effectively return lymph, excess water, waste products and the like accumulated in the legs to the center of body. Further, at this time, due to the Bohr effect achieved by the carbon dioxide absorbed into the legs, muscles and the like of the legs are activated, so that by the synergistic effect, the action of returning the lymph and the like accumulated in the legs to the center of body is enhanced.

Note that when the gas bags 314a to 314h are expanded in order, a large part of the pressurized carbon dioxide in the partition member 312 is discharged to the outside from an insertion opening 317 of the partition member 312.
Therefore, the supply and pressurization control unit 303 repeatedly conducts an operation in which it fills the inside of the partition member 312 with carbon dioxide again, and thereafter, it again supplies gas into the gas bags 314a to 314h.

Note that the supply and pressurization control unit 303 can adjust a strength level of pressurizing the legs by changing an amount of gas supplied to the gas bags 314a to 314h in accordance with the pushing-down of the switch 307. Further, the supply and pressurization control unit 303 can perform adjustment to shorten or lengthen a cycle to pressurize the legs by changing the timing at which the gas is supplied to the gas bags 314a to 314h in accordance with the pushing-down of the switch 307. Further, the supply and pressurization control unit 303 can change the order of expanding the gas bags 314a to 314h and can make a change so that the gas bags 314a to 314h are expanded at a time, in accordance with the pushing-down of the switch 307.

Note that it is also possible that the supply and pressurizing unit 301 used in the tenth embodiment is structured by being divided into a supply unit and a pressurizing unit. Specifically, it is also possible to structure such that the supply unit has only a function of supplying carbon dioxide to the partition member 312, and the pressurizing unit has only a function of supplying gas to the gas bags 314a to 314h. In this case, it is also possible to further structure a control unit controlling the supply unit and the pressurizing unit. Further, the gas supplied by the pressurizing unit may also be air pressurized by a compressor.
Further, a form of the envelope body 310 is not limited to the form of enveloping the entire legs, and may also be a form of enveloping an entire arm, a whole body or the like.
Further, the carbon dioxide supplied by the supply and pressurizing unit 301 or the supply unit may also be the carbon dioxide whose concentration is adjusted by the concentration adjusting unit such as one explained in the eighth embodiment and the ninth embodiment.
Further, in addition to the case of supplying the gas to the plurality of gas bags, the supply and pressurizing unit 301 or the pressurizing unit may also be structured to supply liquid to a plurality of liquid bags, or to pressurize a region of body from above the partition member in the order from the end of body toward the center of body using a roller or the like as solid substance.
Further, in the respective embodiments, the user can appropriately select the absorbent material explained in the aforementioned embodiments or use a plurality of absorbent materials explained in the aforementioned embodiments in a combined manner.

As above, the carbon dioxide supply device of the present invention is structured to pressurize the carbon dioxide supplied into the envelope body to make the carbon dioxide to be absorbed from the body surface. Therefore, a large amount of carbon dioxide supplied into the envelope body is efficiently dissolved in the absorbent material, so that through the absorbent material in which a large amount of carbon dioxide dissolves, the efficiency for making the carbon dioxide to be absorbed into the body surface is improved.

Note that the case where the carbon dioxide supplied into the envelope body is pressurized by using the supply unit and the pressurizing unit as the pressurizing unit of the aforementioned embodiments, is explained, but, the present invention is not limited to this case. For example, it is also possible to generate carbon dioxide through chemical reaction between baking soda and citric acid in the envelope body. In this case, the inside of the envelope body is filled with carbon dioxide generated through the chemical reaction. By further continuously making the chemical reaction occur, the carbon dioxide filled in the envelope body is pressurized.
Further, for example, dry ice may also be included in the envelope body. The dry ice sublimes at room temperature and atmospheric pressure to directly turn into carbon dioxide in a gaseous state. Therefore, the inside of the envelope body is filled with the sublimed carbon dioxide. By further continuously making the chemical reaction occur, the carbon dioxide filled in the envelope body is pressurized. Note that the dry ice is disposed in an isolated manner in the envelope body so that it is not brought into contact with the body surface. As above, as the pressurizing unit, it is also possible to use the chemical reaction to pressurize the carbon dioxide in the envelope body.

Further, it is also possible to structure such that an ultrasonic generator is further provided to the aforementioned carbon dioxide supply device. By applying an ultrasonic wave to the body surface that absorbs carbon dioxide, using the ultrasonic generator, the carbon dioxide can be further efficiently absorbed into the body from the absorbent material.
Further, the aforementioned carbon dioxide supply device may also be structured to supply carbon dioxide to an animal other than the human being. Specifically, the body surface may also be a surface of body of the animal other than the human being.
Further, although a period of time for performing pressurization using the pressurizing unit is different depending on the region of body, the longer the period of time (10 minutes to 30 minutes, for example), the larger the amount of carbon dioxide absorbed from the body surface.
Further, for example, it is also possible to make vitamin which exhibits an effect for recovery from muscle fatigue to be dissolved in the absorbent material in which carbon dioxide can be dissolved. In this case, since the carbon dioxide and the vitamin are absorbed into the body through the absorbent material, the efficiency of the recovery from muscle fatigue is further improved.

### INDUSTRIAL APPLICABILITY

The present invention can be utilized in a cosmetic industry and the like, for example.

## Claims

1. A device for percutaneous absorption of carbon dioxide gas, comprising:
an envelope body enveloping at least a part of body surface;
a supply unit supplying carbon dioxide gas into said envelope body; and
a pressurizing unit pressurizing the carbon dioxide gas supplied into said envelope body to make the gas to be absorbed into an absorbent material.

2. The device for percutaneous absorption of carbon dioxide gas according to claim 1, wherein:
said pressurizing unit is integrally provided with said supply unit; and
said pressurizing unit pressurizes the carbon dioxide gas supplied into said envelope body by using a discharge pressure when discharging the carbon dioxide gas into said envelope body.

3. The device for percutaneous absorption of carbon dioxide gas according to claim 1 or 2, wherein
said supply unit supplies the carbon dioxide gas and the absorbent material into said envelope body.

4. The device for percutaneous absorption of carbon dioxide gas according to claim 1, wherein:
in said envelope body, a partition member partitioning a body surface side and an envelope body side is provided;
said supply unit supplies the carbon dioxide gas to a space formed by the body surface side and the partition member; and
said pressurizing unit pressurizes the carbon dioxide gas supplied to the space formed by the body surface side and the partition member by applying a pressure to the partition member from a space formed by the partition member and said envelope body.

5. The device for percutaneous absorption of carbon dioxide gas according to claim 4, wherein
said pressurizing unit pressurizes the carbon dioxide gas supplied to the space formed by the body surface side and the partition member by supplying gas, liquid or solid substance to the space formed by the partition member and said envelope body.

6. The device for percutaneous absorption of carbon dioxide gas according to claim 4 or 5, wherein
said supply unit supplies the carbon dioxide gas and the absorbent material to the space formed by the body surface side and the partition member.

7. The device for percutaneous absorption of carbon dioxide gas according to claim 1 or 2, wherein:
in said envelope body, there are provided a partition member through which gas can permeate and partitioning a body surface side and an envelope body side, and the absorbent material between the partition member and the body surface; and
said supply unit supplies the carbon dioxide gas to a space formed by the partition member and said envelope body.

8. The device for percutaneous absorption of carbon dioxide gas according to claim 7, wherein
the absorbent material is detachably attached to said envelope body.

9. The device for percutaneous absorption of carbon dioxide gas according to any one of claims 1 to 8, wherein
said envelope body envelopes, out of the body surface, a whole body, an upper half of body, a lower half of body, an arm, a hand, a leg, a femur, a crus, a foot, a neck, a back, a lumbar, a shoulder, a face or a chin.

10. The device for percutaneous absorption of carbon dioxide gas according to any one of claims 1 to 9, wherein
said envelope body has an adhesiveness at least at a contact portion which is brought into contact with the body surface, and uses a material which is resistant to pressurization performed by said pressurizing unit.

11. The device for percutaneous absorption of carbon dioxide gas according to any one of claims 1 to 10, wherein
the material of said envelope body is a material in which a nylon jersey is bonded to a chloroprene rubber.

12. The device for percutaneous absorption of carbon dioxide gas according to any one of claims 1 to 11, wherein
a part of said envelope body has a transparent portion through which an inside of said envelope body can be visually recognized.

13. The device for percutaneous absorption of carbon dioxide gas according to claim 1, wherein
said pressurizing unit pressurizes the carbon dioxide gas supplied into said envelope body by applying a pressure from an outside of said envelope body.

14. The device for percutaneous absorption of carbon dioxide gas according to any one of claims 1 to 13, further comprising
a concentration adjusting unit adjusting a concentration of the carbon dioxide gas supplied into said envelope body by said supply unit.

15. A method for percutaneous absorption of carbon dioxide gas, comprising:
enveloping at least a part of body surface using an envelope body;
supplying carbon dioxide gas into the envelope body; and
pressurizing the carbon dioxide gas supplied into the envelope body to make the gas to be absorbed into an absorbent material.

16. The method for percutaneous absorption of carbon dioxide gas according to claim 15, further comprising
adjusting a concentration of the carbon dioxide gas supplied into the envelope body.

17. An envelope body being an envelope body enveloping at least a part of body surface, comprising
a supply space in which carbon dioxide gas is supplied to an inside thereof, wherein
said envelope body has an adhesiveness at least at a contact portion which is brought into contact with the body surface, and is formed of a material which is resistant to a pressure applied to the carbon dioxide gas supplied to the inside, from the inside or an outside.
